Europäisches Patentamt

European Patent Office

Office européen des brevets

(19)

(11) **EP 1 248 230 A1**

(12) **EUROPEAN PATENT APPLICATION**
published in accordance with Art. 158(3) EPC

(43) Date of publication:
**09.10.2002 Bulletin 2002/41**

(51) Int Cl.[7]: **G06N 3/00**

(21) Application number: **00979977.6**

(22) Date of filing: **07.12.2000**

(86) International application number:
**PCT/JP00/08666**

(87) International publication number:
**WO 02/050767 (27.06.2002 Gazette 2002/26)**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **KYOWA HAKKO KOGYO CO., LTD.**
**Chiyoda-ku, Tokyo 100-8185 (JP)**

(72) Inventors:
• **NISHI, Tatsunari, Kyowa Hakko Kogyo Co. Ltd.**
**Tokyo 100-8185 (JP)**
• **KAWAI, Hironori, Kyowa Hakko Kogyo Co. Ltd.**
**Machida-shi, Tokyo 194-8533 (JP)**

• **IKEMURA, Toshimichi**
**Mishima-shi, Shizuoka 411-0833 (JP)**
• **KUDO, Yoshihiro**
**Yonezawa-shi, Yamagata 992-0062 (JP)**
• **KANAYA, Shigehiko**
**Yonezawa-shi, Yamagata 992-0062 (JP)**
• **KINOUCHI, Makoto**
**Yonezawa-shi, Yamagata 992-0062 (JP)**
• **ABE, Takashi**
**Yamagata-shi, Yamagata 990-0881 (JP)**

(74) Representative: **Casalonga, Axel**
**BUREAU D.A. CASALONGA - JOSSE**
**Paul-Heyse-Strasse 33**
**80336 München (DE)**

(54) **METHOD AND SYSTEM FOR HIGH PRECISION CLASSIFICATION OF LARGE QUANTITY OF INFORMATION DESCRIBED WITH MUTIVARIABLE**

(57)     The present invention provides a method and apparatus for classifying data that can be expressed with multiple variables by similarity using a computer with high accuracy and at high speed, and a method for a computer to execute procedures for classifying data that can be expressed with multiple variables by similarity with high accuracy and high speed, a program for executing the method, and a computer readable recording medium on which is recorded the program. An example of the method comprises the following steps (a) to (f), for classifying input vector data with high accuracy by nonlinear mapping using a computer:

(a) inputting input vector data to a computer,

(b) setting initial neuron vectors,

(c) classifying an input vector into one of the neuron vectors,

(d) updating neuron vectors so as to have a similar structure to structures of input vectors classified into the neuron vector and input vectors classified into the neighborhood of the neuron vector,

(e) repeating step c and step d until a preset number of learning cycles is reached, and

(f) classifying an input vector into one of neuron vectors and outputting.

FIG. 1

(STEP 1) INPUT INPUT VECTER DATA TO A COMPUTER

(STEP 2) SET UP INITIAL NEURON VECTOR $W^0$

(STEP 3) CLASSIFY INPUT VECTOR $x_k$ INTO $W_{p'}$ (PARALLEL PROCESSING POSSIBLE)

(STEP 4) UPDATE $W_{p'}$ (PARALLEL PROCESSING POSSIBLE)

(STEP 5)

LEARNING CYCLE t EQUALS PRESET NUMBER T? NO / YES

(STEP 6) CLASSIFY $x_k$ INTO $W_{p'}$ (PARALLEL PROCESSING POSSIBLE)

EP 1 248 230 A1

**Description**

BACKGROUND OF THE INVENTION

Technical Field

The present invention relates to a method of classifying data that can be expressed with multiple variables by similarity using a computer with high accuracy and at high speed, an apparatus for classifying data that can be expressed with multiple variables by similarity using a computer with high accuracy and at high speed, and a computer readable recording medium on which is recorded a program for a computer to execute procedures for classifying data that can be expressed with multiple variables by similarity with high accuracy and high speed.

Description of the related art

In recent years, with the rapid development of information technology, the amount of data available has become enormous, and the importance of selecting useful information from the data has become greater and greater. In particular, developing a technique that classifies data that can be expressed with multiple variables by similarity using a computer, with high accuracy and at high speed, is an important subject for research and development for selecting and retrieving useful information for industry.

Artificial neural network, which is an engineering field of the neurological sciences, originates in a neuron model proposed by McCulloch and Pins [Bullet. Math. Biophysics, 5, 115-133 (1943)]. The characteristic of this model is that the output of an excitatory/inhibitory state is simplified to 1 or 0, and the state is determined by the sum of stimuli from other neurons. Hebb published a hypothesis (Hebb rule) whereby in a case where transmitted stimuli cause an excitation state in a particular neuron, the connections between neurons that have contributed to the occurrence are enhanced, and the stimuli become easier to transmit [The Organization of Behavior, Wiely, 62. (1949)]. The idea that changes of connection weight bring plasticity to a neural network, leading to memory and learning, is a basic concept of artificial neural networks. Rosenblatt's Perceptron [Psycol. Rev., 65, 6, 386-408 (1958)] is used in various fields of classification problem, since classification can be performed correctly by increasing or decreasing the connection weights of pattern separators.

Self-organizing map: hereunder, abbreviated to SOM) developed by Kohonen, which uses a competitive neural network, is used for recognition of images, sound, fingerprints and the like and the control of production processes of industrial goods ["Application of Self-Organizing Maps — two dimensional visualization of multidimensional information" (Authors: Heizo Tokutaka, Satoru Kishida, Kikuo Fujimura; Kaibundo Publishing Company; first published on July 20th, 1999 ISBN 4-303-73230-3); "Self-Organizing Maps" (Author: T. Kohonen, translated by Heizo Tokutaka, Satoru Kishida, Kikuo Fujimura; Springer-Verlag Tokyo Co., Ltd. Published on June 15th, 1996 ISBN 4-431-70700-X C3055)]. In recent years, as genome information of various organisms has been decoded, a vast amount of information about life has been accumulated, and it is important to solve the secrets of life from this life information using computers in fields such as pharmaceutical development, and the application of SOMs is booming.

The conventional Kohonen's self-organization method (hereunder abbreviated to "conventional method") comprises the following three steps.

Step 1: Initialize a vector on each neuron (referred to hereunder as neuron vector) using a random number.

Step 2: Select the neuron with the closest neuron vector to the input vector.

Step 3: Update the selected neuron and the neighboring neuron vectors.

Step 2 and step 3 are repeated for the number of input vectors. This is defined as one learning cycle, and a specified number of learning cycles is performed. After learning, the input vectors are classified as the neurons having the closest neuron vectors. In Kohonen's SOM, nonlinear mapping can be performed from input vectors in a higher dimensional space to neurons arranged on a lower dimensional map, while maintaining their characteristics.

In this conventional method, since in step 2 and step 3 the updating neuron vectors is performed based on the classification of neuron vectors for each input, a later input vector input later is discriminated more accurately. Therefore, there is a problem in that different self- organizing maps are created depending on the learning order of input vectors. Furthermore, since random numbers are used in the initial neuron vector setting in step 1, the structure of the random numbers influences the self-organizing map obtained after learning. Therefore, there is a problem that factors other than the input vectors are reflected in the self-organizing map. Moreover, there are practical problems whereby in step 1, since random numbers are used, when the initial values differ significantly from the structure of the input vectors, it requires a considerably long learning time, and also in steps 2 and 3, since updating the neuron vectors is performed based on classifying neuron vectors for every input, the learning time becomes longer in proportion to the number of input vectors.

An object of the present invention is to solve the above-described problems. That is to solve:

(1) a problem where, since updating neuron vectors is performed based on classifying neuron vectors for every input in step 2 and step 3, later input vectors are discriminated more accurately, and different self-organizing maps (SOM) are created depending on the learning order of input vectors, so that the same and reproducible SOMs cannot be obtained,

(2) a problem where, since in the conventional method, random numbers are used in the initial neuron vector setting in step 1, the structure of the random numbers influences the SOM obtained after learning, and thus factors other than the input vectors are reflected in the SOM, so that the structure of the input vectors cannot be reflected in the SOM accurately,

(3) a problem where, in the conventional method, since random numbers are used in step 1, when the initial values differ significantly from the structure of the input vectors, a considerably long learning time is required, and

(4) a practical problem where, in the conventional method, since updating of neuron vectors is performed based on classifying neuron vectors for every input in steps 2 and 3, the computing time becomes longer in proportion to the number of input vectors.

SUMMARY OF THE INVENTION

Regarding the problem described above in (1) of being unable to obtain the same and reproducible SOMs, it has been shown that the problem can be solved by designing a batch- processing learning algorithm wherein "the individual neuron vectors are updated after all input vectors are classified into neuron vectors", and applying it to the processing of a sequential processing algorithm in the conventional method, wherein "each input vector is classified into (initial) neuron vectors."

Regarding the problem described above in (2) in which the structure of the input vectors cannot be reflected accurately by a SOM, and the problem in (3) in which a considerably long learning time is required, it has been shown that instead of the method of setting initial neuron vectors using random numbers in the conventional method, the problems can be solved by changing to a method of setting initial neuron vectors by an unsupervised multivariate analysis technique using the distribution characteristics of input vectors of multiple dimensions in multidimensional space, such as principal component analysis, multidimensional scaling or the like.

Furthermore, regarding the practical problem described above in (4) whereby computing time becomes longer in proportion to the number of input vectors, it has been shown that the problem can be solved by applying a batch-learning algorithm instead of the sequential processing algorithm performed in the conventional method, and by parallel learning.

That is, a first embodiment of the present invention is a method comprising the following steps (a) to (f), for classifying input vector data with high accuracy by a nonlinear mapping method using a computer, and the steps are as follows:

(a) inputting input vector data to a computer,

(b) setting initial neuron vectors,

(c) classifying an input vector into one of neuron vectors,

(d) updating neuron vectors so as to have a similar structure to structures of input vectors classified into the neuron vector and input vectors classified into the neighborhood of the neuron vector,

(e) repeating step c and step d until a preset number of learning cycles is reached, and

(f) classifying an input vector into one of neuron vectors and outputting.

In the above-described method, the input vector data may be data of K input vectors (K is a positive integer of 3 or above) of M dimensions (M is a positive integer).

Furthermore, in the above-described method, initial neuron vectors may be set by reflecting the distribution characteristics of input vectors of multiple dimensions in multidimensional space, obtained by an unsupervised multivariate analysis technique, on the arrangement or elements of initial neuron vectors.

For an unsupervised multivariate analysis technique, it is possible to use principal component analysis, multidimensional scaling or the like.

For a method of classifying an input vector into one of neuron vectors, it is possible to use a classification method or the like based on similarity scaling, such as scaling, selected from the group consisting of distance, inner product, and direction cosine.

The above distance may be Euclidean distance or the like.

Furthermore, regarding the classification method in the above embodiment, it is also possible to classify input vectors into neuron vectors using a batch-learning algorithm.

Moreover, using a batch-learning algorithm, it is also possible to update the neuron vectors to a structure similar to structures of the input vectors classified into the neuron vector and input vectors classified into the neighborhood of the neuron vector.

The above processing may be performed using parallel computers.

Another embodiment of the present invention is a method comprising the following steps (a) to (f), for classifying input vector data with high accuracy by a nonlinear mapping method using a computer, and the steps are as follows:

(a) inputting K input vectors (K is a positive integer of 3 or more) $x_k$ (here, k = 1, 2, ..., K) of M dimensions (M is a positive integer) represented by the following equation (1) to a computer,

$$x_k = \{x_{k1}, x_{k2}, ..., x_{kM}\} \tag{1}$$

(b) setting P initial neuron vectors $W^0{}_i$ (here, i = 1, 2, ..., P) arranged in a lattice of D dimensions (D is a positive integer) represented by the following equation (2),

$$W^0{}_i = F\{x_1, x_2, ..., x_K\} \tag{2}$$

(in which, $F\{x_1, x_2, ..., x_k\}$ represents a conversion function for converting from input vectors $\{x_1, x_2, ..., x_K\}$ to initial neuron vectors)

(c) classifying input vectors $\{x_1, x_2, ..., x_K\}$ after t (here, t is the number of the learning cycle, t = 0, 1, 2, ... T) learning cycles into one of P neuron vectors $W^t{}_1, W^t{}_2, ..., W^t{}_P$, arranged in a lattice of D dimensions, using similarity scaling,

(d) for each neuron vector $W^t{}_i$, updating the neuron vector $W^t{}_i$ so as to have a similar structure to structures of the input vectors classified into the neuron vector, and input vectors $x^t{}_1(S_i), x^t{}_2(S_i), ...., x^t{}_{Ni}(S_i)$ classified into the neighborhood of the neuron vector, by the following equation (3),

$$W^{t+1}{}_i = G(W^t{}_i, x^t{}_1(S_i), x^t{}_2(S_i), ...., x^t{}_{Ni}(S_i)) \tag{3}$$

[in which, $x^t{}_n(S_i)$ (n=1, 2, ..., N_i) represents $N_i$ vectors ($N_i$ is the number of input vectors classified into neuron i and neighboring neurons) of M dimensions (M is a positive integer), $W^t{}_{i'}$ represents P neuron vectors (t is the number of learning cycles, i = 1, 2, ..., P) arranged in a lattice of D dimensions (D is a positive integer); when a set of input vectors belonging to the neighboring lattice point to a lattice point were a specific neuron vector $W^t{}_{i'}$ is positioned, $\{x^t{}_1(S_i), x^t{}_2(S_{i'}), ...., x^t{}_N(S_{i'})\}$ is designated as $S_{i'}$, the above equation (3) is an equation to update the neuron vector $W^t{}_{i'}$ to neuron vector $W^{t+1}{}_i$],

(e) repeating step (c) and step (d) until a preset number of learning cycles T is reached, and

(f) classifying the input vectors $\{x_1, x_2, ..., x_K\}$ into one of $W^T{}_1, W^T{}_2, ..., W^T{}_P$ using similarity scaling, and outputting a result.

Another embodiment of the present invention is a method comprising the following steps (a) to (f) for classifying input vector data by nonlinear mapping with high accuracy using a computer, and the steps are as follows:

(a) inputting K (K is a positive integer of 3 or more) input vectors $x_k$ (here, k=1, 2, ..., K) of M dimensions (M is a positive integer) expressed by the following equation (4) to a computer,

$$x_k = \{x_{k1},\, x_{k2},\, \ldots,\, x_{kM}\} \tag{4}$$

(b) setting P (P=I$\times$J) initial neuron vectors $W^0{}_{ij}$ arranged in a two dimensional (*i,j*) lattice (*i*=1, 2, ..., *I*,*j* = 1, 2, ..., *J*) by the following equation (5),

$$W^0{}_{ij} = x_{ave} + 5\sigma_1 \left\{ b_1 \left[ \frac{i - I/2}{I} \right] + b_2 \left[ \frac{j - J/2}{J} \right] \right\} \tag{5}$$

[in which, $x_{ave}$ is the average value of the input vectors, $b_1$ and $b_2$ are the first principal component vector and the second principal component vector respectively obtained by the principal component analysis on the input vectors $\{x_1, x_2, \ldots, x_K\}$, and $\sigma_1$ denotes the standard deviation of the first principal component of the input vectors.]

(c) classifying the input vectors $\{x_1, x_2, \ldots, x_K\}$ after having been through t learning cycles into one of P neuron vectors $W^t{}_1, W^t{}_2, \ldots, W^t{}_P$ arranged in a two-dimensional lattice (t is the number of learning cycles, t = 0, 1, 2, ... T) using similarity scaling,

(d) updating each neuron vector $W^t{}_{ij}$ to $W^{t+1}{}_{ij}$ by the following equations (6) and (7),

$$W^{t+1}{}_{ij} = W^t{}_{ij} + \alpha(t) \left( \frac{\displaystyle\sum_{x_k \in S_{ij}} x_k}{N_{ij}} - W^t{}_{ij} \right) \tag{6}$$

$$\alpha(t) = \max \left\{ 0.01,\, 0.6 \left[ 1 - \frac{t}{T} \right] \right\} \tag{7}$$

[in which, $W^t{}_{ij}$ represents P (P=I$\times$J) neuron vectors arranged on a two dimensional (*i,j*) lattice (*i*=1, 2, ..., *I*,*j* = 1, 2, ..., *J*) after t learning cycles, and the above equation (6) is an equation to update $W^t{}_{ij}$ to $W^{t+1}{}_{ij}$ so as to have asimilar structure to structures of the input vectors ($x_k$) classified into the neuron vector and $N_{ij}$ input vectors $x^t{}_1$ ($S_{ij}$), $x^t{}_2(S_{ij})$, ...., $x^t{}_N(S_{ij})$ classified into the neighborhood of the neuron vector; the term $\alpha(t)$ designates a learning coefficient ($0<\alpha(t)<1$) for epoch t when the number of learning cycles is set to T epochs, and is expressed using a monotone decreasing function.]

(e) repeating step (c) and step (d) until a preset number of learning cycles T is reached, and

(f) classifying the input vectors $\{x_1, x_2, \ldots, x_K\}$ into one of $W^T{}_1, W^T{}_2, \ldots, W^T{}_P$ using similarity scaling, and outputting a result.

The other embodiment of the present invention is a computer readable recording medium on which is recorded a program for performing the method shown in the above-described embodiment, which updates neuron vectors so as to have a similar structure to structures of input vectors classified into the neuron vector and input vectors classified into neighborhoods of the neuron vector.

Here, the program recorded on the recording medium may be a program using a batch-learning algorithm.

Furthermore, the program recorded on the recording medium may be a program for performing the processing of the following equation (8).

$$W^{t+1}{}_i = G(W^t{}_i, x^t{}_1(Si), x^t{}_2(S_i), ...., x^t{}_{Ni}(S_i)) \tag{8}$$

[in which, $x^t{}_k$ (k=1, 2, ..., N) represents K input vectors (K is a positive integer of 3 or more) of M dimensions (M is a positive integer), $W^t{}_i$ represents P neuron vectors (t is the number of learning cycles, i = 1, 2, ..., P) arranged in a lattice of D dimensions (D is a positive integer); when a set of input vectors $\{x^t{}_1(S_i), x^t{}_2(S_i), ...., x^t{}_N(S_i)\}$ belonging to the neighboring lattice point of a lattice point where a specific neuron vector $W^t{}_i$ is positioned equals $S_i$, the above equation (8) is an equation to update the neuron vector $W^t{}_i$ to neuron vector $W^{t+1}{}_i$.]

Furthermore, the program recorded on the recording medium may be a program for performing the processing of the following equations (9) and (10).

$$W^{t+1}{}_{ij} = W^t{}_{ij} + \alpha(t)\left[ \frac{\sum\limits_{x_k \in S_{ij}} x_k}{N_{ij}} - W^t{}_{ij} \right] \tag{9}$$

$$\alpha(t) = \max\left\{ 0.01, 0.6\left[ 1 - \frac{t}{T} \right] \right\} \tag{10}$$

[in which, $W^t{}_{ij}$ represents P (P=I×J) neuron vectors arranged in a two dimensional (i,j) lattice (i=1, 2, ..., I, j = 1, 2, ..., J) after t learning cycles, and the above equation (9) is an equation to update $W^t{}_{ij}$ to $W^{t+1}{}_{ij}$ so as to have a similar structure to structures of input vectors ($x_k$) classified into the neuron vector and $N_{ij}$ input vectors $x^t{}_1(S_{ij}), x^t{}_2(S_{ij}), ...., x^t{}_N(S_{ij})$ classified into the neighborhood of the neuron vectors. The term $\alpha(t)$ designates a learning coefficient ($0 < \alpha(t) < 1$) for the t-th epoch when the number of learning cycles is set to T epochs, and expressed using a monotone decreasing function.]

Furthermore, the abovementioned recording medium may be a computer readable recording medium on which is recorded a program for setting the initial neuron vectors in order to perform the abovementioned method.

Moreover, the recording medium is characterized in that the recorded program is a program for performing the processing of the following equation (11).

$$W^0{}_i = F\{x_1, x_2, ..., x_K\} \tag{11}$$

[in which, $W^0{}_i$ represents P initial neuron vectors arranged in a lattice of D dimensions (D is a positive integer), i is one of 1, 2, ..., P, and $F\{x_1, x_2, ..., x_K\}$ is a function for converting input vectors $\{x_1, x_2, ..., x_K\}$ to K initial neuron vectors.

Furthermore, the recording medium is characterized in that the recorded program is a program for performing the processing of the following equation (12).

$$W^0{}_{ij} = x_{ave} + 5\sigma_l\left\{ b_1\left[ \frac{i - I/2}{I} \right] + b_2\left[ \frac{j - J/2}{J} \right] \right\} \tag{12}$$

[in which, $W^0{}_{ij}$ represents P (P=I×J) initial neuron vectors arranged in a two dimensional (i,j) lattice (i=1, 2, ..., I, j = 1, 2, ..., J), $x_{ave}$ is the average value of K (K is a positive integer of 3 or above) input vectors $\{x_1, x_2, ..., x_K\}$ of M dimensions (M is a positive integer), $b_1$ and $b_2$ are a first principal component vector and a second principal component vector, respectively obtained by principal component analysis on the input vectors $\{x_1, x_2, ..., x_K\}$, and $\sigma_1$ is the standard deviation of the first principal component of the input vectors.]

Furthermore, this may also be a computer readable recording medium characterized in that the recorded program has a program for setting initial neuron vectors for performing the above-described method, and a program for updating neuron vectors to a similar structure to structure of the input vectors classified into the neuron vector and the input vectors classified into the neighborhood of the neuron vector.

Moreover, it may also include a recording medium on which are recorded a program for performing the processing of the following equation (13) and a program for performing the processing of the following equation (14).

$$W^0_i = F\{x_1, x_2, ..., x_K\} \tag{13}$$

(in which, $W^0_i$ represents P initial neuron vectors arranged in a lattice of D dimensions (D is a positive integer), i is one of 1, 2, ..., P, and $F\{x_1, x_2, ..., x_k\}$ is a function for converting from K (K is a positive integer of 3 or above) input vectors of dimension M (M is a positive integer) $\{x_1, x_2, ..., x_K\}$ to initial neuron vectors)

$$W^{t+1}_i = G(W^t_i, x^t_1(S_{i'}), x^t_2(S_{i'}), ...., x^t_N(S_{i'})) \tag{14}$$

[in which, $x^t_n(S_i)$ (n = 1, 2, ..., Ni) represents Ni (Ni is the number of input vectors classified into neuron i and the neighboring neurons) input vectors of M dimensions (M is a positive integer), $W^t_i$, represents P neuron vectors (t is the number of the learning cycle, i=1, 2, ..., P) arranged in a lattice of D dimensions (D is a positive integer), and the above equation (14) is an equation to update $W^t_i$ to $W^{t+1}_i$ such that each neuron vector has a similar structure to structures of the Ni input vectors $x^t_n(S_i)$ classified into the neuron vector].

Furthermore, this is a recording medium on which is recorded a program for performing the processing of the following equations (15), (16) and (17).

$$W^0_{ij} = x_{ave} + 5\sigma_1 \left\{ b_1 \left[ \frac{i - I/2}{I} \right] + b_2 \left[ \frac{j - J/2}{J} \right] \right\} \tag{15}$$

[in which, $W^0_{ij}$ represents P (P=I×J) initial neuron vectors arranged in a two-dimensional $(i,j)$ lattice ($i$=1, 2, ..., I, j = 1, 2, ..., J), $x_{ave}$ is the average value of K (K is a positive integer of 3 or above) input vectors $\{x_1, x_2, ..., x_K\}$ of M dimensions (M is a positive integer), $b_1$ and $b_2$ are the first principal component vector and the second principal component vector respectively obtained by performing principal component analysis on the input vectors $\{x_1, x_2, ..., x_K\}$, and $\sigma_1$ is the standard deviation of the first principal component of the input vectors.]

$$W^{t+1}_{ij} = W^t_{ij} + \alpha(t) \left[ \frac{\sum_{x_k \in S_{ij}} x_k}{N_{ij}} - W^t_{ij} \right] \tag{16}$$

$$\alpha(t) = \max \left\{ 0.01, 0.6 \left[ 1 - \frac{t}{T} \right] \right\} \tag{17}$$

[Here, $W^t_{ij}$ represents P (P=I×J) initial neuron vectors (t is the number of learning cycles, t=1, 2, ..., T) arranged in a two dimensional $(i,j)$ lattice ($i$=1, 2, ..., I,j = 1, 2, ..., J), and the above equation (16) is an equation to update $W^t_{ij}$ to $W^{t+1}_{ij}$ such that each neuron vector has a similar structure to structures of input vectors classified into the neuron

vector and $N_{ij}$ input vectors $x^t{}_n(S_{ij})$ classified into the neighborhood of the neuron vector. The term $\alpha(t)$ denotes a learning coefficient ($0<\alpha(t)<1$) for epoch t when the number of learning cycles is set to T epochs, and expressed using a monotone decreasing function.]

The recording medium on which the abovementioned program is recorded is a recording medium selected from floppy disk, hard disk, magnetic tape, CD-ROM, CD-R, CD-RW, DVD-ROM, DVD-RAM and DVD-RW.

Furthermore, the present embodiment is a computer based system, using the abovementioned computer readable recording medium.

BRIEF DESCRIPTION OF THE DRAWINGS

Fig. 1 is a diagram showing a flow chart of an algorithm of self-organization method of the present invention.

Fig. 2 is a drawing showing the results of creating a SOM in which each gene of sixteen kinds of microorganism is classified by performing principal component analysis using input vectors based on the codon usage frequencies of 29596 genes of sixteen kinds of microorganism to create initial neuron vectors, and by updating the neuron vectors using a method of the present invention. Class numbers of organisms shown in a Table 1 are described for neurons wherein genes of only one species are classified.

Figs. 3A and 3B are drawings showing the result of creating a SOM, wherein initial neuron vectors in which random numbers are used for their initial values are created using input vectors based on the codon usage frequencies of 29596 genes of sixteen kinds of microorganism, and genes for each of the sixteen kinds of microorganism are classified. The results of two independent analyses of creation are shown in Figs. 3A and 3B.

Fig. 4 shows the relationship between number of learning cycles and learning evaluation value when creating a SOM.

Numeral (1) shows the relationship between the number of learning cycles and the learning evaluation value when creating a SOM in which each gene of sixteen kinds of microorganism is classified by performing principal component analysis using input vectors based on the codon usage frequencies of 29596 genes of sixteen kinds of microorganism to create the initial neuron vectors, and by updating the neuron vectors by a method of the present invention.

Numeral (2) shows the relationship between the number of learning cycles and the learning evaluation value when random numbers are used for the initial values instead of performing the principal component analysis in (1).

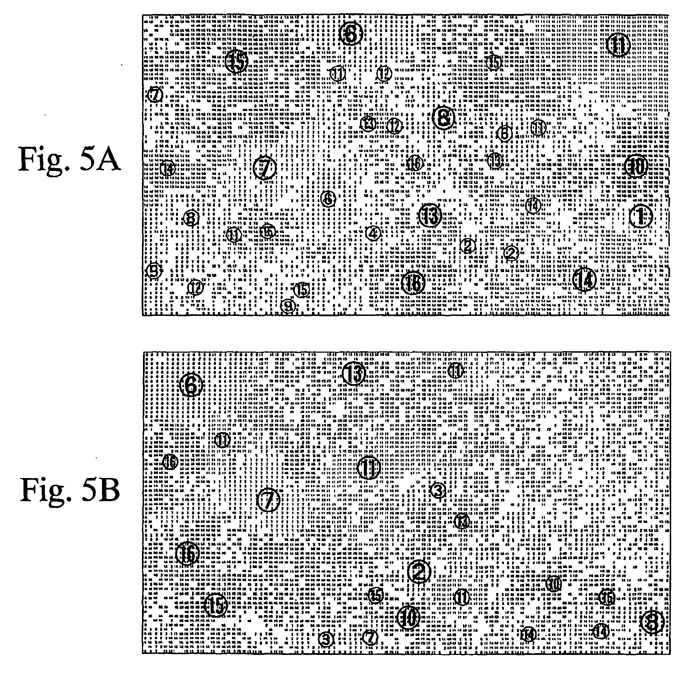

Figs. 5A and 5B show results of creating a SOM, wherein each gene of sixteen kinds of microorganism is classified by performing principal component analysis using input vectors based on the codon usage frequencies of 29596 genes of sixteen kinds of microorganism, to create the initial neuron vectors, and by updating the neuron vectors by the conventional method.

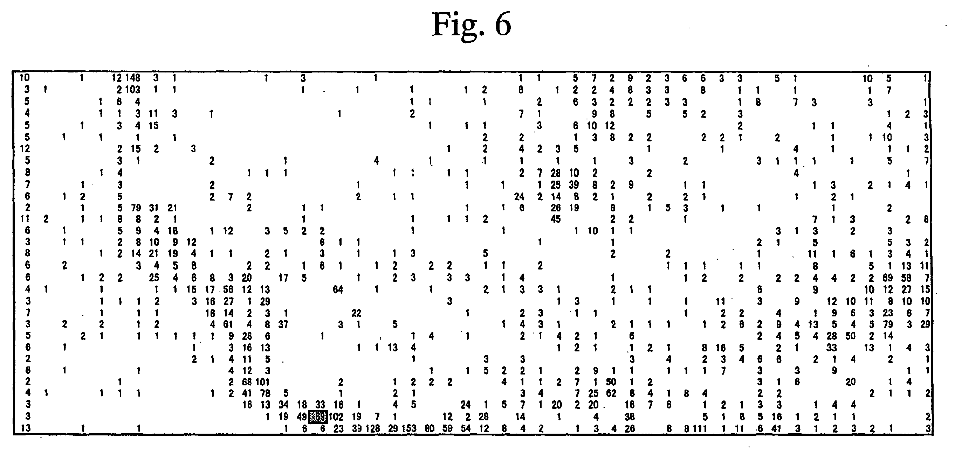

Fig. 6 is a drawing of a SOM created by the method of the present invention using expression level data of 5544 kinds of genes in 60 cancer cell lines. The numbers in the figure denote the numbers of the classified genes.

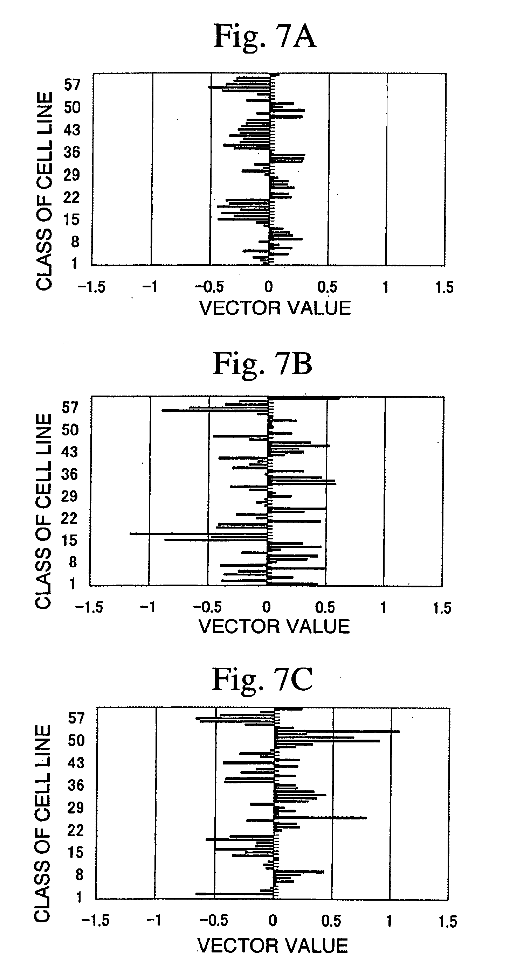

Figs. 7A, 7B, and 7C are drawings showing vector values of neuron vectors of each strain of cancer cell line in a SOM created by the method of the present invention using expression level data of 5544 kinds of genes in 60 cancer cell lines. Fig. 7A represents the vector value of a neuron vector at the position [16, 29] in the SOM, and Figs. 7B and 7C represent the vector values of the neuron vectors of the genes classified at the position [16, 29].

DETAILED DESCRIPTION OF THE INVENTION

As follows is a detailed description of the present invention.

The present invention provides a high accuracy classification method and system using a computer by a nonlinear mapping method having six steps:

(Step 1) inputting input vector data to a computer,

(Step 2) setting initial neuron vectors by a computer,

(Step 3) classifying input an vector into one of neuron vectors by a computer,

(Step 4) updating neuron vectors so as to have a similar structure to structures of input vectors classified into the neuron vector and input vectors classified into the neighborhood of the neuron vectors,

(Step 5) repeating step 3 and step 4 until a preset number of learning cycles is reached, and

(Step 6) classifying input vector into one of neuron vectors and output by a computer.

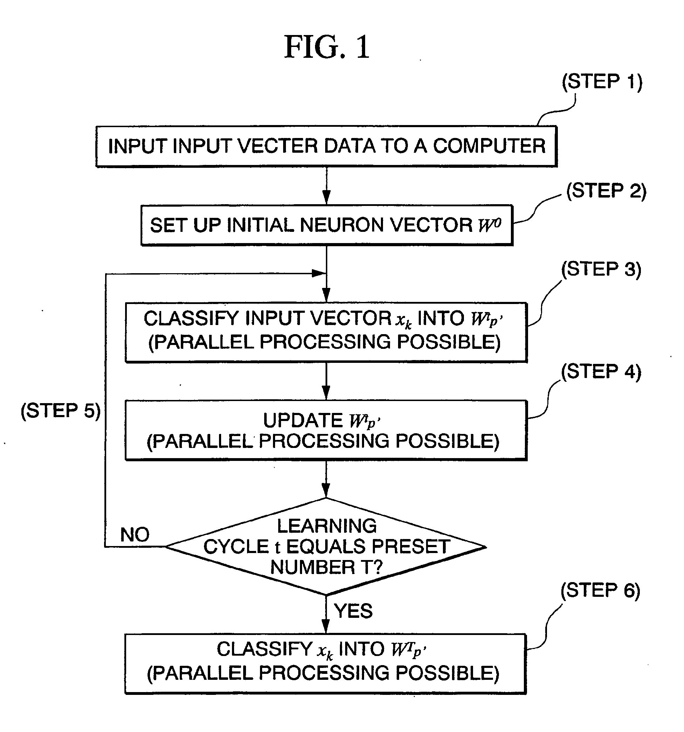

The above steps are shown as a flow chart in Fig. 1.
As follows is a detailed description of each step.

(Step 1)

Input vector data are input to a computer.
For input vector data, any input vector data that are based on data to be analyzed can be used.
Any data that is useful to industry may be used as data to be analyzed.
To be specific, biological data such as nucleotide sequences, amino acid sequences, results of DNA chip analyses and the like, data such as image data, audio data and the like obtained by various measuring instruments, and data such as diagnostic results, questionnaire results and the like can be included.
There are normally K (K is a positive integer of 3 or above) input vectors $\{x_1, x_2, ..., x_K\}$ of M dimensions (M is a positive integer), and each input vector $x_k$ can be represented by the following equation (18).

$$x_k = \{x_{k1}, x_{k2}, ..., x_{kM}\} \tag{18}$$

For k in the equation (1), k = 1, 2, ..., K.
The input vectors are set based on data to be analyzed. Normally, the input vectors are set according to the usual method described in "Application of Self-Organizing Maps — two dimensional visualization of multidimensional information" (Authors: Heizo Tokutaka, Satoru Kishida, Kikuo Fujimura; Kaibundo Publishing Company; first published on July 20th, 1999 ISBN 4-303-73230-3); "Self-Organizing Maps" (Author: T. Kohonen, translated by Heizo Tokutaka, Satoru Kishida, Kikuo Fujimura; Springer-Verlag Tokyo Co., Ltd. Published on June 15th, 1996 ISBN 4-431-70700-X C3055), and the like.
An example of this setting follows.

1) Classification of microorganism genes

In a case where K kinds of gene originating from a plurality of microorganisms are classified, the nucleotide sequence information of these genes is converted such that it can be expressed numerically in M dimensions (64 dimensions in a case where codon usage frequency is used) based on codon usage frequency. Data of M dimensions converted numerically in this manner are used as input vectors.

2) Classification of human genes by expression characteristics

In a case where K kinds of genes originating from human are classified by expression patterns in M kinds of cell lines with different characteristics, the expression levels of these genes in M kindsof cell lines are used as numerical values, and data of M dimensions consisting of the numerical values are set as input vectors.
Step 1 is a step in which input vector data based on information data to be analyzed are input to a computer, and this input can be performed by normal methods, such as manual input, voice input, paper input and the like.

(Step 2)

Initial neuron vectors are set using a computer.
The initial neuron vectors can be set based on random numbers similarly to the conventional method. For the random numbers, random numbers and the like generated on the computer using the C language standard function rand ( ) can be used.
In the case where it is desired that the structure of input vectors is reflected to a SOM accurately, or learning time is shortened, it is preferable to set the initial neuron vectors based on the data of K input vectors $\{x_1, x_2, ..., x_K\}$ of M dimensions set in the above step 1 using a multivariate analysis technique such as principal component analysis, multidimensional scaling and the like, rather than. setting the initial neuron vectors based on random numbers.

In the case where the initial neuron vectors set in this manner consists of a set of P neuron vectors $\{W^0_1, W^0_2, ..., W^0_P\}$ arranged in a lattice of D dimensions (D is a positive integer), each neuron vector can be represented by the following equation (19).

$$W^0_i = F\{x_1, x_2, ..., x_K\} \tag{19}$$

In the equation (2), i = 1, 2, ..., P. Furthermore, $F\{x_1, x_2, ..., x_k\}$ in the equation (2) represents a function for converting from input vectors $\{x_1, x_2, ..., x_K\}$ to initial neuron vectors.

For a specific example, a method of setting the initial neuron vectors in a two-dimensional (D=2) or three-dimensional (D=3) lattice will be described. In accordance with this method, it is possible to set the initial neuron vectors in a lattice of D dimensions.

(1) Method of setting initial neuron vectors in a two dimensional lattice (D=2)

Principal component analysis is performed for K input vectors $\{x_1, x_2, ..., x_K\}$ of M dimensions to obtain a first principal component vector and a second principal component vector, and the obtained principal component vectors are designated $b_1$ and $b_2$, respectively.

Based on these two principal component vectors, principal components $Z_{1k} = b_1 x_k$ and $Z_{2k} = b_2 x_k$ for K input vectors are obtained (k=1, 2, ..., K). The standard deviations of $\{Z_{11}, Z_{12}, ..., Z_{1k}, ..., Z_{1K}\}$ and $\{Z_{21}, Z_{22}, ..., Z_{2k}, ..., Z_{2K}\}$ are designated $\sigma_1$ and $\sigma_2$ respectively.

The average value of the input vectors is obtained, and the average value obtained is designated $x_{ave}$.

Two-dimensional lattice points with two dimensions are represented by $ij(i = 1, 2, ..., I, j = 1, 2, ..., J)$, and neuron vectors $W^0_{ij}$ are placed at the two-dimensional lattice points $(ij)$. The values of I and J may be integers of 3 or above. Preferably, J is the largest integer less than $I \times \sigma_2 / \sigma_1$. The value of I may be set appropriately depending on the number of input vector data. In general a value of 50 to 1000 is used, and typically a value of 100 is used.

$W^0_{ij}$ can be defined by an equation (20).

$$W^0_{ij} = x_{ave} + 5\sigma_I \left\{ b_1 \left[ \frac{i - I/2}{I} \right] + b_2 \left[ \frac{j - J/2}{J} \right] \right\} \tag{20}$$

(2) Method of setting initial neuron vectors in a three-dimensional lattice (D=3)

In the principal component analysis in (1) described above, a third principal component vector is obtained in addition to the first principal component vector and the second principal component vector, and the obtained first, second and third principal component vectors are designated $b_1$, $b_2$ and $b_3$ respectively.

Based on these three principal components, principal components $Z_{1k} = b_1 x_k$, $Z_{2k} = b_2 x_k$, and $Z_{3k} = b_3 x_k$ are obtained. The standard deviations of $\{Z_{11}, Z_{12}, ..., Z_{1k}, ..., Z_{1K}\}$, $\{Z_{21}, Z_{22}, ..., Z_{2k}, ..., Z_{2K}\}$ and $\{Z_{31}, Z_{32}, ..., Z_{3k}, ..., Z_{3K}\}$ are designated $\sigma_1$, $\sigma_2$, and $\sigma_3$ respectively. Three-dimensional lattice points are represented by $ijl(i = 1, 2, ..., I, j = 1, 2, ..., J, l = 1, 2, ..., L)$, and neuron vectors $W^0_{ijl}$ are placed at the three-dimensional lattice points $(ijl)$. The values of I, J and L may be integers of 3 or above. Preferably, J and L are the largest integers less than $I \times \sigma_2 / \sigma_1$ and $I \times \sigma_3 / \sigma_1$ respectively. The value of I may be set appropriately depending on the number of input vector data. In general a value of 50 to 1000 is used, and typically a value of 100 is used.

$W^0_{ijl}$ can be defined by an equation (21).

$$W^0_{ijl} = x_{ave} + 5\sigma_I \left\{ b_1 \left[ \frac{i - I/2}{I} \right] + b_2 \left[ \frac{j - J/2}{J} \right] + b_3 \left[ \frac{l - L/2}{L} \right] \right\} \tag{21}$$

(Step 3)

All of the input vectors $\{x_1, x_2, ..., x_K\}$ are classified into neuron vectors.

To be specific, after all of the input vectors $\{x_1, x_2, ..., x_K\}$ t learning cycles using similarity scaling (distance, inner product, direction cosine or the like), they are each classified as one of P neuron vectors $W^t{}_1, W^t{}_2, ..., W^t{}_P$ using a computer.

Here, t is the number of the learning cycle (epoch). In the case of T learning cycles, t = 0, 1, 2, ... T. The I-th neuron vector at the t-th epoch t can be represented by $W^t{}_i$. Here, i = 1, 2, ..., P.

The neuron vectors of the value of t = 0 correspond to the initial neuron vectors set in step 2.

Classification of each input vector $x_k$ can be performed by calculating the Euclidean distance to each neuron vector $W^t{}_i$, and classifying the input vector into the neuron vector having the smallest Euclidean distance. Here, in the case of a neuron vector located at a two-dimensional lattice point ($ij$), $W^t{}_i$ can be represented by $W^t{}_{ij}$.

The input vectors $\{x_1, x_2, ..., x_K\}$ may be classified into $W^t{}_i$ by parallel processing for each input vector $x_k$.

(Step 4)

For each neuron vector $W^t{}_i$, the neuron vector $W^t{}_i$ is updated so as to have a similar structure to structures of input vectors ($x_k$) classified into the neuron vector and input vectors classified into the neighborhood of the neuron vector.

That is to say, a set of input vectors belonging to a lattice point at which a specific neuron vector $W^t{}_{i'}$ is positioned is designated $S_{i'}$. The neuron vectors $W^t{}_{i'}$ ($i'$ = 1, 2,..., P) are updated by obtaining new neuron vectors ($W^{t+1}{}_{i'}$) that reflect the structure of input vectors belonging to $S_{i'}$ from N vectors $x^t{}_1(S_{i'}), x^t{}_2(S_{i'}), ...., x^t{}_N(S_{i'})$ belonging to $S_{i'}$ and $W^t{}_{i'}$, using function G in the following equation (22).

$$W^{t+1}{}_{i'}=G(W^t{}_{i'}, x^t{}_1(S_{i'}), x^t{}_2(S_{i'}), ...., x^t{}_N(S_{i'})) \tag{22}$$

For a specific example, updating of a neuron vector $W^t{}_{ij}$ set on a two-dimensional lattice will be described. A neuron vector set on a lattice of D dimensions may be updated in the same manner.

When an input vector $x_k$ is belonging to a neuron vector $W^t{}_{ij}$ arranged in a two-dimensional lattice, and a set of input vectors belonging to neighboring lattice points of the lattice point at which $W^t{}_{ij}$ is positioned is designated $S_{ij}$, it is possible to update the neuron vector $W^t{}_{ij}$ by obtaining a new neuron vector $W^{t+1}{}_{ij}$ that reflects the structure of input vectors belonging to $S_{ij}$ from $N_{ij}$ input vectors $x^t{}_1(S_{ij}), x^t{}_2(S_{ij}), ...., x^t{}_{Nij}(S_{ij})$ belonging to $S_{ij}$ and $W^t{}_{ij}$, by the following equation (23).

$$W^{t+1}{}_{ij}=W^t{}_{ij}+\alpha(t)\left[\frac{\displaystyle\sum_{x_k \in S_{ij}} x_k}{N_{ij}} - W^t{}_{ij}\right] \tag{23}$$

Here, $N_{ij}$ is the total number of input vectors classified into $S_{ij}$.

The term $\alpha(t)$ designates a learning coefficient ($0<\alpha(t)<1$) for epoch t when the number of learning cycles is set to T epochs, and uses a monotone decreasing function. Preferably, it can be obtained by the following equation (24).

The number of learning cycles T may be set appropriately depending on the number of input vector data. In general it is set between 10 epochs and 1000 epochs, and typically 100 epochs.

$$\alpha(t)=\max\left\{0.01, 0.6\left[1-\frac{t}{T}\right]\right\} \tag{24}$$

The neighboring set $S_{ij}$ is a set of input vectors $x_{ij}$ classified as lattice points $i'j'$ which satisfy the conditions

of $i - \beta(t) \leq i' \leq i + \beta(t)$ and $j - \beta(t) \leq j' \leq j + \beta(t)$. The symbol $\beta(t)$ represents the number that determines the neighborhood, and is obtained by an equation (25).

$$\beta(t) = \max\{0, 25\text{-}t\} \tag{25}$$

It is possible to update the neuron vector $\{W^t{}_1, W^t{}_2, ..., W^t{}_P\}$ by parallel processing for each neuron vector $W^t{}_i$.

(Step 5)

Learning is performed by repeating step 3 and step 4 until the preset number of epochs T is reached.

(Step 6)

After learning is completed, corresponding to the method in step 3, input vectors $x_k$ are classified into neuron vectors $W^T{}_i$ by a computer, and the results are output.

Based on the classification reference represented by $W^T{}_i$, in which the structure of the input vectors is reflected, the input vectors $x_k$ are classified. That is, in the case where a plurality of input vectors are classified as the same neuron vector, it is clear that the vector structures of these input vectors are very similar.

It is possible to classify the input vectors $\{x_1, x_2, ..., x_K\}$ by parallel processing for each input vector $x_k$.

The output of the classification result by the above-described steps may be visualized by displaying it as a SOM.

Creation and display of a SOM may be performed according to the method described in "Application of Self-Organizing Maps — two dimensional visualization of multidimensional information" (Authors: Heizo Tokutaka, Satoru Kishida, Kikuo Fujimura; Kaibundo Publishing Company; first published on July 20th, 1999 ISBN 4-303-73230-3); "Self-Organizing Maps" (Author: T. Kohonen, translated by Heizo Tokutaka, Satoru Kishida, Kikuo Fujimura; Springer-Verlag Tokyo Co., Ltd. Published on June 15th, 1996 ISBN 4-431-70700-X C3055), and the like.

For example, the classification results of input vectors obtained by placing neuron vectors at two-dimensional lattice points can be displayed as a two-dimensional SOM using "Excel" spreadsheet software from Microsoft Corporation or the like. To be specific, after applying a suitable label to each lattice point based on the characteristics of the input vectors belonging to each lattice point of neuron vectors having two-dimensional lattice points, these label values are exported to Excel, and using the functions of Excel, these labels can be displayed as a SOM on a monitor, printed or the like in a two-dimensional lattice. It is also possible that the values of the total number of input vectors belonging to each lattice point are exported to Excel, and these values of the total number are displayed as a SOM on a monitor, printed or the like in a two-dimensional lattice using the functions of Excel.

For a computer to use in the above-described steps, anything can be used as long as it has the functions of a computer. However, it is preferable to use one with a fast calculation speed. A specific example is a SUN Ultra 60 workstation manufactured by Sun Microsystems Inc. and the like. The above steps 1 to 6 do not need to be performed using the same computer. That is, it is possible to output a result obtained in one of the above steps to another computer, and process the succeeding step in the other computer.

Furthermore, it is also possible to perform the computational processing of the steps (steps 3, 4, 5 and 6), for which parallel processing is possible, in parallel using a computer with multiple CPUs or a plurality of computers. In the conventional method, a sequential learning algorithm is used, so parallel processing is not possible. However, in the present invention, a batch-learning algorithm is used, so parallel processing is possible.

Since parallel processing is possible, the computing time required to classify input vectors can be shortened considerably.

That is, if the times to process the above 6 steps using one processor are T1, T2, T3/C, T4/C, T5/C and T6 respectively, and parallel processing is performed by C processors, ideally the times required by each step become T1, T2, T3, T4, T5 and T6 respectively, and the total computing time can be shortened by

T1+T2+T3+T4+T5+T6 -{T1+T2+(T3+T4+T5)/C+T6}

=(1-1/C)(T3+T4+T5)

The steps 2 to 6 can be automated by using a computer readable recording medium, on which a program for performing the procedure from steps 2 to 6 is recorded. The recording medium is a recording medium of the present invention.

"Computer readable recording medium" means any recording medium that a computer can read and access directly. Such a recording medium may be a magnetic storage medium such as floppy disk, hard disk, magnetic tape and the like, an optical storage medium such as CD-ROM, CD-R, CD-RW, DVD-ROM, DVD-RAM, DVD-RW and the like, an electric storage medium such as RAM, ROM and the like, or a hybrid (for example, a magnetic/optical storage medium such as MO) of these categories. However, it is not limited to these.

The computer based system, wherein the above-described computer readable recording medium of the present invention is used, is a system of the present invention.

"Computer based system" means one comprising a hardware device, a software device and a data storage device, which are used for analyzing information stored on a computer readable recording medium of the present invention.

The hardware device basically comprises an input devices, a data storage device, a central processing unit and an output device.

Software device means a device which uses a program for a computer to perform the procedures from steps 2 to 6 stored on the recording medium of the present invention.

Data storage device means memory to store input information and calculation results, and a memory access device that can access it.

That is to say, a computer based system of the present invention is characterized in that there is provided:

(i) an input device for inputting input vector data;

(ii) a software device for processing the input data using a program for the computer to perform steps 2 to 6; and

(iv) an output device for outputting classification results obtained by the software device in (iii).

As follows are examples of the present invention.

EXAMPLES

Example 1

For each of 29596 genes of 16 kinds of microorganism described in the Table 1, principal component analysis based on input vectors based on the codon usage frequency of the gene is performed, using a SUN Ultra 60 workstation manufactured by Sun Microsystems Inc. to create initial neuron vectors, and a SOM is created. A DNA sequence data array for each gene was obtained from ftp://ncbi.nlm.nih.gov/genbank/genomes/bacteria/.

As follows is a detailed description.

Table 1

| Training set used for development of neuron vectors | | | | |
|---|---|---|---|---|
| Name of Organism | Abbreviation | Number of Genes | Class | ID number |
| *Archaeoglobus fulgidus* | AFU | 2088 | 1 | 1- 2088 |
| *Aquifex aeolicus* | AAE | 1489 | 2 | 2089- 3577 |
| *Borrelia burgdorferi* | BBU | 772 | 3 | 3578- 4349 |
| *Bacillus subtilis* | BSU | 3788 | 4 | 4350- 8137 |
| *Chlamydia trachomatis* | CTR | 833 | 5 | 8138- 8970 |
| *Escherichia coli* | ECO | 3913 | 6 | 8971-12883 |
| *Helicobacter pylori* | HPY | 1392 | 7 | 12884-14275 |
| Haemophilus influenzae | HIN | 1572 | 8 | 14276-15847 |
| *Methanococcus jannashii* | MJA | 1522 | 9 | 15848-17369 |
| *Methanobacterium thermoautotrophicum* | MTH | 1646 | 10 | 17370-19015 |
| *Mycobacterium tuberculosis* | MTU | 3675 | 11 | 19016-22690 |
| *Mycoplasma genitalium* | MGE | 450 | 12 | 22691-23140 |

Table 1   (continued)

| Training set used for development of neuron vectors | | | | |
|---|---|---|---|---|
| Name of Organism | Abbreviation | Number of Genes | Class | ID number |
| *Mycoplasma pneumoniae* | MPN | 657 | 13 | 23141-23797 |
| *Pyrococcus horikoshii* | PHO | 1973 | 14 | 23798-25770 |
| *Synechocystis sp.* | SYN | 2909 | 15 | 25771-28679 |
| *Treponema pallidum* | TPA | 917 | 16 | 28680-29596 |
| Total number of genes: 29596 | | | | |

(a) Calculation of Input Vectors and Setting of Initial Neuron Vectors

For genes of each microorganism in Table 1, each gene is given an ID number as shown in Table 1 such that *Archcaeoglobus fulgidus*, AF ODU1 gene is the first gene, and *Treponema pallidum,* TP1041 gene is the 29596th gene.
For all of the genes, the frequency of each of 64 kinds of codons in a translated region from translation start codon to termination codon is obtained according to the codon number table in Table 2. A vector comprising the codon frequency $C_{km}$ (m = 1, 2, ..., 64) of gene k is designated $C_k = (C_{k1}, C_{k2}, ..., C_{k64},)$.
To be specific, for *Escherichia coli thrA* gene (8971st gene) in Table 1, for example, the number of the 1st codon (Phe) is 11, the 2nd codon (Phe) is 19, the 3rd codon (Leu) is 10, the 4th codon (Leu) is 13, the 5th codon (Ser) is 11, the 6th codon (Ser) is 10, the 7th codon (Ser) is 6, the 8th codon (Ser) is 9, the 9th codon (Tyr) is 12, the 10th codon (Tyr) is 8, the 11th codon (Ter) is 0, the 12th codon (Ter) is 0, the 13th codon (Cys) is 3, the 14th codon (Cys) is 9, the 15th codon (Ter) is 1, the 16th codon (Trp) is 4, the 17th codon (Leu) is 8, the 18th codon (Leu) is 13, the 19th codon (Leu) is 2, the 20th codon (Leu) is 43, the 21st codon (Pro) is 3, the 22nd codon (Pro) is 6, the 23rd codon (Pro) is 2, the 24th codon (Pro) is 18, the 25th codon (His) is 8, the 26th codon (His) is 6, the 27th codon (Gln) is 11, the 28th codon (Gln) is 19, the 29th codon (Arg) is 18, the 30th codon (Arg) is 19, the 31st codon (Arg) is 3, the 32nd codon (Arg) is 4, the 33rd codon (Ile) is 30, the 34th codon (Ile) is 15, the 35th codon (Ile) is 1, the 36th codon (Met) is 23, the 37th codon (Thr) is 5, the 38th codon (Thr) is 19, the 39th codon (Thr) is 2, the 40th codon (Thr) is 8, the 41st codon (Asn) is 22, the 42nd codon (Asn) is 16, the 43rd codon (Lys) is 22, the 44th codon (Lys) is 12, the 45th codon (Ser) is 3, the 46th codon (Ser) is 12, the 47th codon (Arg) is 0, the 48th codon (Arg) is 2, the 49th codon (Val) is 19, the 50th codon (Val) is 18, the 51st codon (Val) is 5, the 52nd codon (Val) is 27, the 53rd codon (Ala) is 15, the 54th codon (Ala) is 36, the 55th codon (Ala) is 14, the 56th codon (Ala) is 26, the 57th codon (Asp) is 30, the 58th codon (Asp) is 14, the 59th codon (Glu) is 40, the 60th codon (Glu) is 13, the 61st codon (Gly) is 22, the 62nd codon (Gly) is 22, the 63rd codon (Gly) is 9, the 64th codon (Gly) is 1, so the codon usage frequency vector of the gene can be expressed as $C_{8971}=$ (11, 19, 10, 13, 11, 10, 6, 9, 12, 8, 0, 0, 3, 9, 1, 4, 8, 13, 2, 43, 3, 6, 2, 18, 8, 6, 11, 19, 18, 19, 3, 4, 30, 15, 1, 23, 5, 19, 2, 8, 22, 16, 22, 12, 3, 12, 0, 2, 19, 18, 5, 27, 15, 36, 14, 26, 30, 14, 40, 13, 22, 22, 9, 10).

Table 2

| Codon number table | | | | | |
|---|---|---|---|---|---|
| First Letter | Second Letter | | | | Third Letter |
| | T | C | A | G | |
| T | 1(Phe) | 5(Ser) | 9(Tyr) | 13(Cys) | T |
| | 2(Phe) | 6(Ser) | 10(Tyr) | 14(Cys) | C |
| | 3(Leu) | 7(Ser) | 11(Ter*) | 15(Ter*) | A |
| | 4(Leu) | 8(Ser) | 12(Ter*) | 16(Trp) | G |
| C | 17(Leu) | 21(Pro) | 25(His) | 29(ArG) | T |
| | 18(Leu) | 22(Pro) | 26(His) | 30(ArG) | C |
| | 19(Leu) | 23(Pro) | 27(Gln) | 31(ArG) | A |
| | 20(Leu) | 24(Pro) | 28(Gln) | 32(ArG) | G |

Ter*: termination codon

Table 2   (continued)

| Codon number table | | | | | |
|---|---|---|---|---|---|
| First Letter | Second Letter | | | | Third Letter |
| | T | C | A | G | |
| A | 33(Ile) | 37(Thr) | 41(Asn) | 45(Ser) | T |
| | 34(Ile) | 38(Thr) | 42(Asn) | 46(Ser) | C |
| | 35(Ile) | 39(Thr) | 43(Lys) | 47(ArG) | A |
| | 36(MeT) | 40(Thr) | 44(Lys) | 48(ArG) | G |
| G | 49(Val) | 53(AlA) | 57(Asp) | 61(Gly) | T |
| | 50(Val) | 54(AlA) | 58(Asp) | 62(Gly) | C |
| | 51(Val) | 55(AlA) | 59(Glu) | 63(Gly) | A |
| | 52(Val) | 56(AlA) | 60(Glu) | 64(Gly) | G |

When the codon usage frequency vector of gene ID $k$ determined by the above method is $C_k$, input vector $x_k$ ={$x_{k1}$, $x_{k2}$, ..., $x_{kM}$} ($M$=64) of gene ID $k$ can be calculated by the following equation (8). For m here, m= 1, 2, ..., 64.

$$x_{km} = \frac{C_{km}}{\sum_{n=1}^{M} C_{kn}}$$

(9)

To be specific, in gene *thrA* of *Escherichia coli thrA* gene of codon usage frequency vector $C_{8971}$=(11, 19, 10, 13, 11, 10, 6, 9, 12, 8, 0, 0, 3, 9, 1, 4, 8, 13, 2, 43, 3, 6, 2, 18, 8, 6, 11, 19, 18, 19, 3, 4, 30, 15, 1, 23, 5, 19, 2, 8, 22, 16, 22, 12, 3, 12, 0, 2, 19, 18, 5, 27, 15, 36, 14, 26, 30, 14, 40, 13, 22, 22, 9, 10), the input vectors become $x_{8971}$ = (0.0134, 0.0231, 0.0122, 0.0158, 0.0134, 0.0122, 0.0073, 0.0110, 0.0146, 0.0097, 0.0000, 0.0000, 0.0037, 0.0110, 0.0012, 0.0049, 0.0097, 0.0158, 0.0024, 0.0524, 0.0037, 0.0073, 0.0024, 0.0219, 0.0097, 0.0073, 0.0134, 0.0231, 0.0219, 0.0231, 0.0037, 0.0049, 0.0365, 0.0183, 0.0012, 0.0280, 0.0061, 0.0231, 0.0024, 0.0097, 0.0268, 0.0195, 0.0268, 0.0146, 0.0037, 0.0146, 0.0000, 0.0024, 0.0231, 0.0219, 0.0061, 0.0329, 0.0183, 0.0438, 0.0171, 0.0317, 0.0365, 0.0171, 0.0487, 0.0158, 0.0268, 0.0268, 0.0110, 0.0122).

A first principle component vector $b_1$ and a second principal component $b_2$ are obtained by performing principal component analysis for input vectors based on all of the 29596 kinds of genes, which are created by the above method. The results are shown as follows.
$b_1$= (-0.1876, 0.0710, -0.2563, -0.0100, -0.0778, 0.0400, -0.0523, 0.0797, -0.1245, 0.0254, -0.0121, 0.0013, -0.0203, 0.0228, 0.0025, 0.0460, -0.0727, 0.0740, -0.0353, 0.2936, -0.0470, 0.0686, -0.0418, 0.1570, -0.0229, 0.0582, -0.0863, 0.1070, 0.0320, 0.1442, 0.0208, 0.1180, -0.2116, 0.1070, -0.1550, 0.0048, -0.0676, 0.1536, -0.0664, 0.0607, -0.1962, 0.0128, -0.3789, -0.0720, -0.0492, 0.0331, -0.0975, -0.0176, -0.1183, 0.1423, -0.0577, 0.1757, -0.0690, 0.2778, -0.0260, 0.2361, -0.1318, 0.1606, -0.2148, 0.0412, 0.0260, 0.2081, -0.0757, 0.0506)
$b_2$= (0.1525, 0.1048, -0.1891, -0.1399, -0.0539, 0.0112, 0.0281, -0.0246, -0.0922, 0.1455, -0.0059, 0.0001, -0.0215, 0.0134, 0.0062, -0.0386, 0.0938, 0.1603, -0.0026, -0.0785, -0.0104, 0.0285, 0.0181, -0.0550, -0.0719, 0.0243, -0.2403, -0.0425, -0.1203, -0.1199, -0.0351, -0.0518, -0.1903, -0.0411, 0.3417, 0.0179, -0.0391, -0.0644, 0.0178, -0.0177, -0.1515, 0.0320, -0.1318, 0.3510, -0.0449 -0.0138, 0.1530, 0.3162, 0.1676, 0.0160, 0.0342, -0.0725, -0.0221, -0.0656, 0.0271, -0.1325, -0.0695, 0.0942, -0.0141, 0.4003, -0.0403, -0.1298, 0.1701, 0.0146)
Here, the standard deviations $\sigma_1$ and $\sigma_2$ of the first and second principal components of the two input vectors are 0.05515 and 0.03757 respectively, and the average $x_{ave}$ of the input vectors is $x_{ave}$= (0.0266, 0.0167, 0.0217, 0.0169, 0.0105, 0.0096, 0.0098, 0.0071, 0.0170, 0.0148, 0.0020, 0.0008, 0.0051, 0.0058, 0.0015, 0.0114, 0.0175, 0.0151, 0.0078, 0.0242, 0.0098, 0.0104, 0.0096, 0.0125, 0.0105, 0.0094, 0.0170, 0.0168, 0.0091, 0.0115, 0.0038, 0.0073, 0.0309, 0.0215, 0.0170, 0.0235, 0.0106, 0.0169, 0.0119, 0.0108, 0.0205, 0.0182, 0.0378, 0.0235, 0.0093, 0.0129, 0.0104, 0.0108, 0.0228, 0.0148, 0.0125, 0.0230, 0.0189, 0.0237, 0.0190, 0.0207, 0.0296, 0.0202, 0.0403, 0.0278, 0.0178, 0.0210, 0.0177, 0.0139)

Next, two-dimensional lattice points are represented as $ij(i = 1, 2, ..., I, j = 1, 2, ..., J)$, and 64- dimensional neuron vectors $W^0_{ij}=(w^0_{ij1}, w^0_{ij2}, ..., w^0_{ij64})$ are placed at the two-dimensional lattice points ($ij$). Here $I$ is 100, and $J$ is the largest integer less than $I \times \sigma_2/\sigma_1$. In the present analysis, $J$ turned out to be 68. $W^0_{ij}$ is defined by an equation (27).

$$W^0_{ij}=x_{ave}+5\sigma_l\left\{ b_l\left[\frac{i-I/2}{I}\right] + b_2\left[\frac{j-J/2}{J}\right]\right\} \tag{27}$$

(b) Classification of Neuron Vectors

Input vectors $x_k$ based on each of 29596 kinds of genes are classified into neuron vectors $W^t_{ij}$ with the smallest Euclidean distances.

(c) Update of Neuron Vectors

Next, the neuron vectors $W^t_{ij}$ are updated by the following equation (28).

$$W^{t+1}_{ij}=W^t_{ij}+\alpha(t)\left(\frac{\sum_{x_k\in S_{ij}} x_k}{N_{ij}} - W^t_{ij}\right) \tag{28}$$

The learning coefficient $\alpha(t)$ ($0<\alpha(t)<1$) for the t-th epoch when the number of learning cycles is set to T epochs is obtained by an equation (29). The present experiment is performed with 100 learning cycles (100 epochs).

$$\alpha(t)=\max\left\{0.01, 0.6\left[1-\frac{t}{T}\right]\right\} \tag{29}$$

The neighboring set $S_{ij}$ is a set of input vectors $x_k$ classified as lattice points $i'j'$ which satisfy the conditions of $i - \beta(t) \le i' \le i + \beta(t)$ and $j - \beta(t) \le j' \le j + \beta(t)$. Furthermore, $N_{ji}$ is the total of vectors classified into $S_{ij}$. $\beta(t)$ represents a number that determines the neighborhood, and is obtained by an equation (30).

$$\beta(t) = \max\{0, 25-t\} \tag{30}$$

(d) Learning Process

Subsequently, the above steps (b) and (c) are repeated 100 (=T) times.

Here, the learning is evaluated by square error as defined by the following equation (31).

$$Q^t=\sum_{n=1}^{N} |x_k - W^t_{ij(k)}|^2$$

$$\tag{31}$$

Here, N (=29596) is the total number of genes, and $W^t_{ij(k)}$ is the neuron vector with the smallest Euclidean distance from $x_k$. The interpretation of this learning evaluation value Q is that the smaller the value, the better the information of the input vectors is reflected on the neuron vectors.

(e) Classification of Input Vectors into Neuron Vectors

The input vectors $x_k$ based on each of the 29596 kinds of genes are classified into neuron vectors $W^T_{ij(k)}$ obtained as a result of 100 epochs of learning cycles with the smallest Euclidean distance.

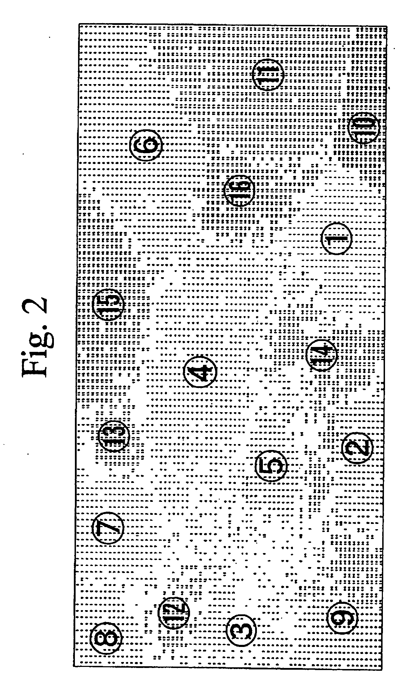

An SOM obtained by the classification is shown in Fig. 2. In a case where a gene originating from only one kind of microorganism is classified, the class number of the microorganism from which the gene originated is displayed in Table 1. For the SOM in Fig. 2, it is shown that exactly the same map as in Fig. 2 is obtained even if it is recreated, and a reproducible map can be created.

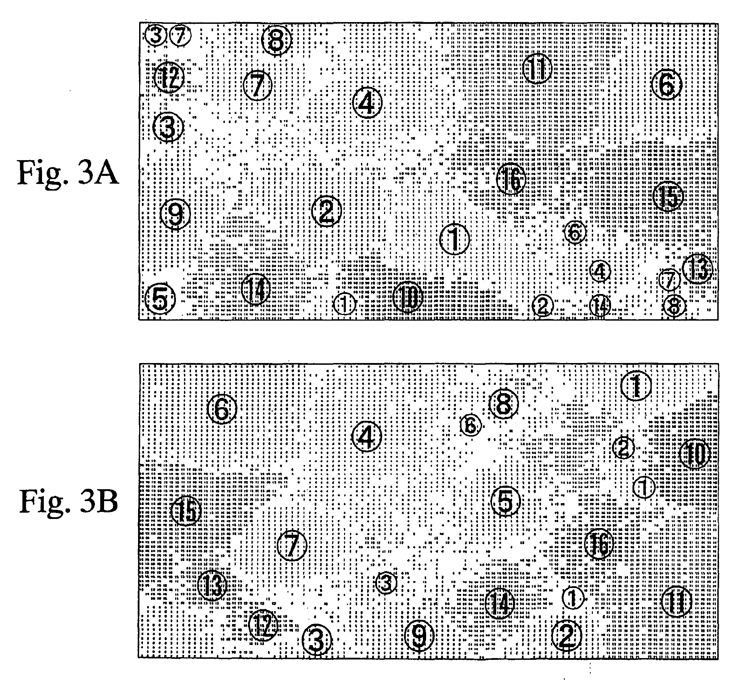

Neuron vectors $W^0_{ij}$ were defined using random numbers for analysis without performing principal component analysis for input vectors, and the result is shown in a reference example 1 described later. However, the results are different for each analysis in the conventional method, and the same and reproducible result could not be obtained (refer to Fig. 3A and Fig. 3B).

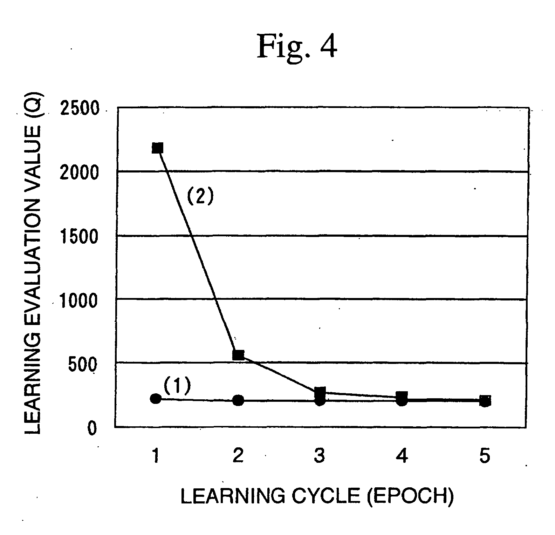

Furthermore, "the relationship between the number of learning cycles (epochs) and learning evaluation value Q" is shown in Fig. 4. By using a method of the present invention wherein principal component analysis is used for initial value setting, it has been shown that input vector data can be reflected in neuron vectors better in fewer learning cycles than in the case where initial values are set by random numbers as described in the reference example 1, that is to say, a shortening of calculation time and improvement in classification accuracy can be achieved.

Updating of neuron vectors was performed by a sequential processing algorithm of the conventional method for analysis, and the results are shown in a reference example 2 described later. However, in the conventional method, a different SOM was created depending on the input order of input vectors $x_k$, and the degree of grouping was very low.

As described above, according to the present invention, it has been shown that it is possible to achieve the same and reproducible analysis result (SOM) independent of the input order of input vectors, in a short time and with high accuracy.

Reference Example 1

The classification analysis of genes of each of 16 kinds of microorganism was performed by the same method as in Example 1 except that neuron vectors $W^0_{ij}$ were defined using random numbers without performing principal component analysis for input vectors in step 1 — (a) in the above-described Example 1.

Neuron vector $W^t_{ij}$ was defined by the following method.

Random numbers were generated by using the C language standard function rand( ) within a range between a minimum value $\min_{k=1,2,...,29596} (x_{km})$ and a maximum value $\max_{k=1,2...,29596} (x_{km})$ for each of the m-th (m=1, 2, ..., 64) variable of input data $x_k = \{x_{k1}, x_{k2}, ..., x_{k64}\}$ (k=1, 2, ..., 29596) obtained by the above-described Example 1 — (a). Neuron vectors $W^0_{ij}$ were defined by the following equation (32).

Here, $W^0_{ij} = (W^0_{ij1}, W^0_{ij2}, ..., W^0_{ijm}, ..., W^0_{ij64})$.

$$W^0_{ijm} = \min_{k=1,2,...,29596} (x_{km}) +$$

$$\{\max_{k=1,2,...,29596} (x_{km}) - \min_{k=1,2,...,29596} (x_{km})\}\mathrm{rand}()/2147483647 \tag{32}$$

After defining $W^0_{ij}$, genes of each of 16 kinds of microorganism were classified according to the method in Example 1. The same analysis was repeated.

Results of the two analyses are shown in Figs. 3A and 3B. Furthermore, "the relationship between the number of learning cycles (epochs) and learning evaluation value Q" in the first analysis is shown in Fig. 4.

As shown in Figs. 3A and 3B, when random numbers are used, completely different SOMs are created for each analysis, and the degree of grouping is lower than in the case where principal component analysis as shown in embodiment 1 is performed.

Reference Example 2

The classification analysis of genes of each of 16 kinds of microorganism was performed by performing classification and updating of neuron vectors in steps (b) and (c) in the above-described Example 1 using the following equation (33) instead of the equation (28).

$$W^{t+1}{}_{ij(k)} = W^t{}_{ij(k)} + \alpha(t)(x_k - W^t{}_{ij(k)}) \tag{33}$$

That is, neuron vectors $W^t{}_{ij(k)}$ were updated using the above equation (33) each time an input vector was classified in step (b) of Example 1.

A learning coefficient $\alpha(t)$ ($0<\alpha(t)<1$) for the t-th epoch when the number of learning cycles is set to T epochs was obtained by the equation (29). The present experiment was performed with the number of learning cycles being 100 (100 epochs).

$$\alpha(t) = \max\left\{0.01, 0.6\left[1 - \frac{t}{T}\right]\right\} \tag{34}$$

The neighboring neuron vectors of $W^t{}_{ij(k)}$ were also updated according to the equation (33) at the same time as $W^t{}_{ij(k)}$. The neighborhood $S_{ij}$ is a set of neuron vectors at lattice points $i'j'$ which satisfy the conditions of $i - \beta(t) \leq i' \leq i + \beta(t)$ and $j - \beta(t) \leq j' \leq j + \beta(t)$. B(t) represents a number that determines the neighborhood, and is obtained by the equation (35).

$$\beta(t) = \max\{0, 25-t\} \tag{35}$$

Neuron vectors were updated by inputting, in order, from input vector $x_1$ of a gene whose ID number is 1 in Table 1 to input vector $x_{29596}$ of a gene whose ID number is 29596, and updating from $W^{t+1}{}_{ij(1)}$ to $W^{t+1}{}_{ij(29596)}$ in order.

The SOM obtained is shown in Fig. 5A.

The same analysis was performed in the reverse order of updating the neuron vectors $W_{ij(k)}$. That is, updating was performed from $W^{t+1}{}_{ij(29596)}$ to $W^{t+1}{}_{ij(1)}$ in order followed by inputting, in order, from input vector $x_{29596}$ of a gene whose ID number is 29596 in Table 1 to input vector $x_1$ of a gene whose ID number is 1.

The SOM obtained is shown in Fig. 5B.

The degree of grouping is very low, and in the SOM created in the input order from ID number 1, genes originating from microorganisms of ID numbers 3 (*Borrelia burgdorferi*), and 5 (*Chlamzdia trachomatis*) were not grouped at all. Furthermore, in the SOM created in the input order from ID number 29596, genes originating from ID numbers 1 (*Archaeoglobus fulgidus*), 4 (*Bacillus subtilis*), 5 (*Chlamzdia trachomatis*), 9 (*Methanococcus jannascii*) and 12 (*Mycoplasma genitalium*) were not grouped at all. It has been shown that different SOMs are created depending on the input order of data, so it is not appropriate to use the present method to interpret data wherein the input order is meaningless.

Example 2

[Analysis of Gene Expression Levels and Classification of Genes in Cancer Cell Lines]

Data from the results of measuring the expression level of each gene in 60 strains of cancer cell using a DNA microarray, which is described in "A Gene Expression Database for the Molecular Pharmacology of Cancer" in Nature Genetics, <u>24</u>, 236-244 (2000) (Uwe Scherf et al.) were analyzed using a method of the present invention. The 60 cancer cell lines are shown in Tables 3-1 and 3-2. Here, the data was obtained as "all_genes.txt" from a web page "http://discover.nci.nih.gov/nature2000/" opened by the authors of this paper.

Of 10009 genes included in the present file, excluding those genes having description of "NA" or "—INF", cDNAs of 5544 human genes were used for analysis.

The analysis of the data was performed based on the method in embodiment 1.

Table 3-1

| Cell lines used for analysis by DNA microarray | | |
|---|---|---|
| Abbreviation of Cell Strain | Name of Cell Strain | Class |
| ME:LOXIMVI | Melanoma line | 1 |
| ME:MALME-3M | Melanoma line | 2 |
| ME:SK-MEL-2 | Melanoma line | 3 |
| ME:SK-MEL-5 | Melanoma line | 4 |
| ME:SK-MEL-28 | Melanoma line | 5 |
| LC:NCI-H23 | Non-small-cell lung cancer cells | 6 |
| ME:M14 | Melanoma line | 7 |
| ME:UACC-62 | Melanoma line | 8 |
| LC:NCI-H522 | Non-small-cell lung cancer cells | 9 |
| LC:A549/ATCC | Non-small-cell lung cancer cells | 10 |
| LC:EKVX | Non-small-cell lung cancer cells | 11 |
| LC:NCI-H322M | Non-small-cell lung cancer cells | 12 |
| LC:NCI-H460 | Non-small-cell lung cancer cells | 13 |
| LC:HOP-62 | Non-small-cell lung cancer cells | 14 |
| LC:HOP-92 | Non-small-cell lung cancer cells | 15 |
| CNS:SNB-19 | CNS lines | 16 |
| CNS:SNB-75 | CNS lines | 17 |
| CNS:U251 | CNS lines | 18 |
| CNS:SF-268 | CNS lines | 19 |
| CNS:SF-295 | CNS lines | 20 |
| CNS:SF-539 | CNS lines | 21 |
| CO:HT29 | Colon cancer lines | 22 |
| CO:HCC-2998 | Colon cancer lines | 23 |
| CO:HCT-116 | Colon cancer lines | 24 |
| CO:SW-620 | Colon cancer lines | 25 |
| CO:HCT-15 | Colon cancer lines | 26 |
| CO:KM12 | Colon cancer lines | 27 |
| OV:OVCAR-3 | Ovarian lines | 28 |
| OV:OVCAR-4 | Ovarian lines | 29 |
| OV:OVCAR-8 | Ovarian lines | 30 |

Table 3-2

| Cell strains used for analysis by DNA microarray | | |
|---|---|---|
| Abbreviation of Cell Strain | Name of Cell Strain | Class |
| OV:IGROV1 | Ovarian lines | 31 |
| OV:SK-OV-3 | Ovarian lines | 32 |

Table 3-2   (continued)

| Cell strains used for analysis by DNA microarray | | |
|---|---|---|
| Abbreviation of Cell Strain | Name of Cell Strain | Class |
| LE:CCRF-CEM | Leukamia | 33 |
| LE:K-562 | Leukamia | 34 |
| LE:MOLT-4 | Leukamia | 35 |
| LE:SR | Leukamia | 36 |
| RE:UO-31 | Renal carcinoma lines | 37 |
| RE:SN12C | Renal carcinoma lines | 38 |
| RE:A498 | Renal carcinoma lines | 39 |
| RE:CAKI-1 | Renal carcinoma lines | 40 |
| RE:RXF-393 | Renal carcinoma lines | 41 |
| RE:786-0 | Renal carcinoma lines | 42 |
| RE:ACHN | Renal carcinoma lines | 43 |
| RE:TK-10 | Renal carcinoma lines | 44 |
| ME:UACC-257 | Melanoma line | 45 |
| LC:NCI-H226 | Non-small-cell lung cancer cells | 46 |
| CO:COLO205 | Colon cancer lines | 47 |
| OV:OVCAR-5 | Ovarian lines | 48 |
| LE:HL-60 | Leukamia | 49 |
| LE:RPMI-8226 | Leukamia | 50 |
| BR:MCF7 | Breast origin | 51 |
| BR:MCF7/ADF-RES | Breast origin | 52 |
| PR:PC-3 | | 53 |
| PR:DU-145 | | 54 |
| BR:MDA-MB-231/ATCC | Breast origin | 55 |
| BR:HS578T | Breast origin | 56 |
| BR:MDA-MB-435 | Breast origin | 57 |
| BR:MDA-N | Breast origin | 58 |
| BR:BT-549 | Breast origin | 59 |
| BR:T-47D | Breast origin | 60 |

(a) Calculation of Input Vectors and Setting of Initial Neuron Vectors

The above 5544 human genes are numbered (k=1, 2,..., 5544) in order, and input vectors $x_k=\{x_{k1},x_{k2}, ..., x_{km}\}$, are set using data of the expression level of each gene in 60 cancer cell lines (m=1, 2,..., 60).

A first principle component vector $b_1$ and a second principal component $b_2$ are obtained by performing principal component analysis on the 5544 input vectors defined. The results are shown as follows.

$b_1$= (0.0896, 0.1288, 0.1590, 0.1944, 0.1374, 0.1599, 0.1391, 0.1593, 0.1772, 0.0842, 0.0845, 0.0940, 0.1207, 0.0914, 0.1391, 0.0940, 0.0572, 0.0882, 0.1192, 0.0704, 0.0998, 0.1699, 0.1107, 0.1278, 0.1437, 0.1381, 0.1116, 0.0640, 0.0538, 0.0983, 0.1086, 0.1003, 0.2140, 0.1289, 0.2224, 0.2147, 0.0781, 0.1618, 0.0762, 0.0641, 0.0682, 0.0859, 0.0785, 0.0933, 0.1465, 0.0294, 0.1315, 0.1068, 0.1483, 0.1227, 0.1654, 0.1059, 0.0872, 0.1158, 0.1877, 0.0316, 0.2129, 0.2098, 0.0892, 0.1450)

$b_2$= (-0.0521, -0.1201, -0.1072, -0.0397, -0.1300, 0.0219, -0.1011, -0.1356, 0.0482, -0.0195, -0.0520, 0.0434, -0.0207, -0.1006, -0.1727, -0.1212, -0.1955, -0.1003, -0.1803, -0.1443, -0.1692, 0.1880, 0.1319, 0.0643, 0.1701, 0.1315, 0.1240, 0.0642, 0.0044, -0.0946, 0.0218, -0.0408, 0.2394, 0.2236, 0.2652, 0.1047, -0.1363, -0.1330, -0.1089, -0.1011, -0.1618, -0.1027, -0.1120, -0.0943, -0.0458, -0.0980, 0.2100, -0.0138, 0.2235, 0.1251, 0.1935, -0.0711, 0.0296, -0.0203, -0.1285, -0.2642, -0.1032, -0.0809, -0.1166, 0.0994)

Here, the standard deviations $\sigma_1$ and $\sigma_2$ of the first principal component value and second principal component value of the 5544 input vectors are 3.3367 and 2.0720 respectively, and $x_{ave}$ the average of the input vectors is $x_{ave}$= (-0.0164, -0.0157, -0.0306, 0.0043, -0.0529, 0.0730, -0.0421, 0.0132, 0.0020, -0.0544, -0.0592, 0.0192, -0.0320, 0.0513, -0.0712, -0.0336, -0.0131, 0.0170, -0.1138, -0.1020, 0.0504, -0.1454, 0.0255, -0.0727, 0.0164, 0.0704, 0.0579, 0.0140, -0.0322, 0.0588, -0.0390, 0.0878, -0.0175, -0.1021, -0.1015, -0.0833, 0.0137, -0.1347, -0.0009, 0.0424, 0.0168, -0.0164, -0.0243, 0.0203, -0.0417, 0.0220, -0.0592, -0.0317, -0.0372, -0.1114, -0.1365, 0.0383, 0.0142, 0.0608, -0.1329, -0.0718, -0.1357, -0.0276, -0.0131, 0.0022).

Next, two-dimensional lattice points are represented by $ij$ ($i$ = 1, 2, ..., $I$, $j$ = 1, 2, ..., $J$), and 60- dimensional neuron vectors $W^0_{ij}$=($w^0_{ij1}$, $w^0_{ij2}$, ..., $w^0_{ij64}$) are placed at the two-dimensional lattice points ($ij$). Here $I$ is 50, and $J$ is the largest integer less than $I \times \sigma_2/\sigma_1$. In the present analysis, $J$ turned out to be 31. $W^0_{ij}$ is defined by an equation (16).

$$W^0_{ij}=x_{ave}+5\sigma_1\left\{ b_1\left[\frac{i-I/2}{I}\right] + b_2\left[\frac{j-J/2}{J}\right]\right\} \tag{36}$$

(b) Classification of Neuron Vectors

Next, all of the 5544 input vectors $x_k$ are classified into neuron vectors $W^t_{ij}$ with the smallest Euclidean distance. Neuron vectors into which $x_k$ are classified are represented by $W^t_{ij(k)}$.

(c) Update of Neuron Vectors

Next, the neuron vectors $W^t_{ij}$ are updated by the following equation (37).

$$W^{t+1}_{ij}=W^t_{ij}+\alpha(t)\left(\frac{\displaystyle\sum_{x_k\in S_{ij}}x_k}{N_{ij}} - W^t_{ij}\right) \tag{37}$$

The learning coefficient $\alpha(t)$ ($0<\alpha(t)<1$) for epoch t when the number of learning cycles is set to T epochs is obtained by an equation (38).

$$\alpha(t)=\max\left\{0.01, 0.6\left[1 - \frac{t}{T}\right]\right\} \tag{38}$$

The neighboring set $S_{ij}$ is a set of input vectors $x_{ij}$ classified as lattice points $i'j'$ which satisfy the conditions of $i - \beta(t) \le i' \le i + \beta(t)$ $and$ $j - \beta(t) \le j' \le j + \beta(t)$. Furthermore, $N_{ij}$ is the total number of vectors classified into $S_{ij}$. The symbol $\beta(t)$ represents the number that determines the neighborhood, and is obtained by an equation (39).

$$\beta(t) = \max\{0, 10-t\} \tag{39}$$

(d) Learning Process

Next, the above steps (b) and (c) are repeated 100 (=T) times.

Here, the learning effectiveness for the t-th epoch is evaluated by square error as defined by the following equation (40).

$$Q^t = \sum_{n=1}^{N} |x_k - W^t{}_{ij(k)}|^2$$

(40)

Here, N is the total number of genes, and $W^t{}_{ij(k)}$ is a neuron vector with the smallest Euclidean distance from $x_k$.

(e) Classification of Input Vectors into Neuron Vectors

All of the 5544 input vectors $x_k$ are classified into neuron vectors $W^T{}_{ij(k)}$ obtained as a result of 100 epochs of learning cycles with the smllest Euclidean distance. An SOM obtained by the classification is shown in Fig. 6. The numbers of genes classified into each neuron are shown in Fig. 6.

In Fig. 7, neuron vectors (Fig. 7A) at a position [16, 29] and all vectors (Figs. 7A, 7B and 7C) of genes (genes encoded in colons of EST entries in GenBank Accession Nos. T55183 and T54809 and genes encoded in EST clones of Accession Nos. W76236 and W72999) classified are shown as a bar chart. This figure clarifies that Figs. 7B and 7C, and Fig. 7A show very similar vector patterns.

That is to say, it has been shown that genes whose expression patterns are almost the same in human cells can be classified into the same neuron vectors by a method of the present invention.

INDUSTRIAL APPLICABILITY

According to the present invention, it is possible to create the same and reproducible self-organizing map using an enormous amount of input data, and classify and obtain useful information with high accuracy. Furthermore, calculation processing time can be shortened considerably.

**Claims**

1. A method for classifying input vector data with high accuracy by a nonlinear mapping method using a computer, which comprises the following steps (a) to (f):

   (a) inputting input vector data to a computer,

   (b) setting initial neuron vectors,

   (c) classifying an input vector into one of neuron vectors,

   (d) updating neuron vectors so as to have a similar structure to structures of input vectors classified into the neuron vector and input vectors classified into the neighborhood of the neuron vector,

   (e) repeating step c and step d until a preset number of learning cycles is reached, and

   (f) classifying an input vector into one of neuron vectors and outputting.

2. The method according to claim 1, wherein the input vector data are data of K input vectors (K is a positive integer of 3 or above) of M dimensions (M is a positive integer).

3. The method according to claim 1, wherein the initial neuron vectors are set by reflecting on the arrangement or elements of the initial neuron vectors, the distribution characteristics of input vectors of multiple dimensions in multidimensional space, obtained by an unsupervised multivariate analysis technique.

**4.** The method according to claim 3, wherein the unsupervised multivariate analysis technique is the principal component analysis or the multidimensional scaling.

**5.** The method according to claim 1, wherein the classifying an input vector into one of neuron vectors is performed based on the similarity scaling selected from the group consisting of scaling of distance, inner product, and direction cosine.

**6.** The method according to claim 5, wherein the distance is a Euclidean distance.

**7.** The method according to claim 1 or 6, wherein the classifying an input vectors into one of neuron vectors is performed using a batch-learning algorithm.

**8.** The method according to claim 1, wherein the updating neuron vector so as to have a similar structure to structures of input vectors classified into the neuron vector and input vectors classified into the neighborhood of the neuron vector, is performed using a batch-learning algorithm.

**9.** The method according to claim 7 or 8, wherein the method is performed using parallel computers.

**10.** A method of classifying input vector data with high accuracy using a computer, by a nonlinear mapping method, which comprises the following steps (a) to (f):

(a) inputting K (K is a positive integer of 3 or above) input vectors $x_k$ ( k = 1, 2, ..., K) of M dimensions (M is a positive integer) represented by the following equation (1) to a computer,

$$x_k = \{x_{k1}, x_{k2}, ..., x_{kM}\} \qquad (1)$$

(b) setting P initial neuron vectors $W^0_i$ (here, i = 1, 2, ..., P) arranged in a lattice of D dimensions (D is a positive integer) represented by the following equation (2),

$$W^0_i = F\{x_1, x_2, ..., x_K\} \qquad (2)$$

(in which, F$\{x_1, x_2, ..., x_k\}$ represents a function for converting from input vectors $\{x_1, x_2, ..., x_K\}$ to initial neuron vectors)

(c) classifying the input vectors $\{x_1, x_2, ..., x_K\}$ after t (t is the number of the learning cycle, t = 0, 1, 2, ... T) learning cycles into one of P neuron vectors $W^t_1, W^t_2, ..., W^t_P$, arranged in a lattice of D dimensions, using similarity scaling,

(d) for each neuron vector $W^t_i$, updating the neuron vector $W^t_i$ so as to have a similar structure to structures of input vectors classified into the neuron vector, and input vectors
$x^t_1(S_i), x^t_2(S_i), ...., x^t_{Ni}(S_i)$ classified into the neighborhood of the neuron vector, by the following equation (3),

$$W^{t+1}_i = G(W^t_i, x^t_1(S_i), x^t_2(S_i), ...., x^t_{Ni}(S_i)) \qquad (3)$$

[in which, $x^t_n(S_i)$ (n=1, 2, ..., $N_i$) represents $N_i$ vectors ($N_i$ is the number of input vectors classified into neuron i and neighboring neurons) with M dimensions (M is a positive integer), $W^t_i$ represents P neuron vectors (t is the number of learning cycles, i = 1, 2, ..., P) arranged in a lattice of D dimensions (D is a positive integer); when a set of input vectors associated with the neighboring lattice point to a lattice point where a specific neuron vector $W^t_i$ is positioned, $\{x^t_1(S_i), x^t_2(S_i), ...., x^t_N(S_i)\}$ equals $S_i$, the above equation (3) is an equation to update the neuron vector $W^t_i$ to neuron vector $W^{t+1}_i$].

(e) repeating step (c) and step (d) until a preset number of learning cycles T is reached, and

(f) classifying the input vectors $\{x_1, x_2, ..., x_K\}$ into one of $W^T_1, W^T_2, ..., W^T_P$ using similarity scaling, and outputting a result.

**11.** A method of classifying input vector data with high accuracy using a computer, by a nonlinear mapping method, which comprises the following steps (a) to (f):

(a) inputting K (K is a positive integer of 3 or above) input vectors $x_k$ (here, k=1, 2, ..., K) of M dimensions (M is a positive integer) expressed by the following equation (4) to a computer,

$$x_k = \{x_{k1}, x_{k2}, ..., x_{kM}\} \tag{4}$$

(b) setting P (P=I×J) initial neuron vectors $W^0_{ij}$ arranged in a two-dimensional (i,j) lattice (i=1, 2, ..., I, j = 1, 2, ..., J) by the following equation (5),

$$W^0_{ij} = x_{ave} + 5\sigma_I \left\{ b_I \left[ \frac{i - I/2}{I} \right] + b_2 \left[ \frac{j - J/2}{J} \right] \right\} \tag{5}$$

[in which, $x_{ave}$ represents the average value of the input vectors, $b_1$ and $b_2$ are the first principal component vector and the second principal component vector respectively obtained by the principal component analysis on the input vectors $\{x_1, x_2, ..., x_K\}$, and $\sigma_1$ denotes the standard deviation of the first principal component of the input vectors.]

(c) classifying the input vectors $\{x_1, x_2, ..., x_K\}$ after t learning cycles, into one of P neuron vectors $W^t_1, W^t_2, ..., W^t_P$ arranged in a two-dimensional lattice (t is the number of learning cycles, t = 0, 1, 2, ... T) using similarity scaling,

(d) updating each neuron vector $W^t_{ij}$ to $W^{t+1}_{ij}$ by the following equations (6) and (7),

$$W^{t+1}_{ij} = W^t_{ij} + \alpha(t) \left[ \frac{\sum_{x_k \in S_{ij}} x_k}{N_{ij}} - W^t_{ij} \right]. \tag{6}$$

$$\alpha(t) = \max \left\{ 0.01, 0.6 \left[ 1 - \frac{t}{T} \right] \right\} \tag{7}$$

[in which, $W^t_{ij}$ represents P (P=I×J) neuron vectors arranged on a two-dimensional (i,j) lattice (i=1, 2, ..., I, j = 1, 2, ..., J) after t learning cycles, and the above equation (6) is an equation to update $W^t_{ij}$ to $W^{t+1}_{ij}$ so as to have a similar structure to structures of input vectors ($x_k$) classified into the neuron vector and $N_{ij}$ input vectors $x^t_1(S_{ij}), x^t_2(S_{ij}), ...., x^t_N(S_{ij})$ classified into the neighborhood of the neuron vector; the term $\alpha(t)$ designates a learning coefficient ($0<\alpha(t)<1$) for the t-th epoch when the number of learning cycles is set to T epochs, and is expressed using a monotone decreasing function.]

(e) repeating step (c) and step (d) until a preset number of learning cycles T is reached, and

(f) classifying the input vectors $\{x_1, x_2, ..., x_K\}$ into one of $W^T_1, W^T_2, ..., W^T_P$ using similarity scaling, and outputting a result.

**12.** A computer readable recording medium on which is recorded a program for performing the method according to any one of claims 1 to 11, which updates neuron vectors so as to have a similar structure to structures of input vectors classified into the neuron vector and input vectors classified into the neighborhood of neuron vector.

**13.** The recording medium according to claim 12, wherein said program is a program using a batch-learning algorithm.

**14.** The recording medium according to claim 12 or 13, wherein said program is a program for performing the processing of the following equation (8):

$$W^{t+1}_i = G(W^t_i, x^t_1(S_i), x^t_2(S_i), ...., x^t_{Ni}(S_i)) \tag{8}$$

[in which, $x^t_k$ (k=1, 2, ..., N) represents K input vectors (K is a positive integer of 3 or above) of M dimensions (M is a positive integer), $W^t_i$ represents P neuron vectors (t is the number of learning cycles, i = 1, 2, ..., P) arranged in a lattice of D dimensions (D is a positive integer); when a set of input vectors associated with the neighboring lattice point to a lattice point where a specific neuron vector $W^t_i$ is positioned $\{x^t_1(S_i), x^t_2(S_i), ...., x^t_{Ni}(S_i)\}$ is designated as $S_i$, the above equation (8) is an equation to update the neuron vector $W^t_i$ to neuron vector $W^{t+1}_i$.]

**15.** The recording medium according to claim 12 or 13, wherein said program is a program for performing the processing of the following equations (9) and (10):

$$W^{t+1}_{ij} = W^t_{ij} + \alpha(t)\left(\frac{\sum_{x_k \in S_{ij}} x_k}{N_{ij}} - W^t_{ij}\right) \tag{9}$$

$$\alpha(t) = \max\left\{0.01, 0.6\left[1 - \frac{t}{T}\right]\right\} \tag{10}$$

[in which, $W^t_{ij}$ represents P (P=I×J) neuron vectors arranged in a two-dimensional (i,j) lattice (i=1, 2,..., I, j = 1,2, ..., J) after t learning cycles, and the above equation (9) is an equation to update $W^t_{ij}$ to $W^{t+1}_{ij}$ so as to have a similar structure to structures of input vectors ($x_k$) classified into the neuron vector and $N_{ij}$ input vectors $x^t_1(S_{ij})$, $x^t_2(S_{ij})$, ...., $x^t_N(S_{ij})$ classified into the neighborhood of the neuron vectors; the term $\alpha(t)$ designates a learning coefficient ($0 < \alpha(t) < 1$) for the t-th epoch when the number of learning cycles is set to T epochs, and is expressed using a monotone decreasing function.]

**16.** A computer readable recording medium on which is recorded a program for setting the initial neuron vectors in order to perform the method according to any one of claims 1 to 11.

**17.** The recording medium according to claim 16, wherein said program is a program for performing the process of following equation (11):

$$W^0_i = F\{x_1, x_2, ..., x_K\} \tag{11}$$

[in which, $W^0_i$ represents P initial neuron vectors arranged in a lattice of D dimensions (D is a positive integer), i is one of 1, 2, ..., P, and F$\{x_1, x_2, ..., x_k\}$ is a function for converting input vectors $\{x_1, x_2, ..., x_K\}$ to initial neuron vectors.

**18.** The recording medium according to claim 16, wherein said program is a program for performing the processing of the following equation (12):

$$W^0_{ij} = x_{ave} + 5\sigma_I \left\{ b_I \left[ \frac{i - I/2}{I} \right] + b_2 \left[ \frac{j - J/2}{J} \right] \right\} \tag{12}$$

[in which, $W^0_{ij}$ represents P (P=I×J) initial neuron vectors arranged in a two-dimensional (*i,j*) lattice (*i*=1, 2, ..., *I,j* = 1,2, ..., *J*), $x_{ave}$ is the average value of K (K is a positive integer of 3 or above) input vectors {$x_1$, $x_2$, ..., $x_K$} of M dimensions (M is a positive integer), $b_1$ and $b_2$ are a first principal component vector and a second principal component vector respectively obtained by principal component analysis on the input vectors {$x_1$, $x_2$, ..., $x_K$}, and $\sigma_1$ is the standard deviation of the first principal component of the input vectors.]

**19.** A computer readable recording medium on which is recorded a program for setting initial neuron vectors for performing the method according to any one of claims 1 to 11, and a program for updating neuron vectors so as to have a similar structure to structures of input vectors classified into the neuron vector and input vectors classified into the neighborhood of the neuron vector.

**20.** The recording medium according to claim 19, wherein the program is a program for performing the processing of the following equations (13) and (14):

$$W^0_i = F\{x_1, x_2, ..., x_K\} \tag{13}$$

(in which, $W^0_i$ represents P initial neuron vectors of D dimensions (D is a positive integer) arranged in a lattice, i is one of 1, 2, ..., P, and F{$x_1$, $x_2$, ..., $x_K$} is a function for converting from K (K is a positive integer of 3 or above) input vectors {$x_1$, $x_2$, ..., $x_K$} of M dimensions (M is a positive integer) to initial neuron vectors)

$$W^{t+1}_i = G(W^t_i, x^t_1(S_i), x^t_2(S_i), ...., x^t_N(S_i)) \tag{14}$$

[in which, $x^t_n(S_i)$ (n = 1, 2, ..., Ni) represents Ni (Ni is the number of input vectors classified into neuron i and the neighboring neurons) input vectors of M dimensions (M is a positive integer), $W^t_i$, represents P neuron vectors (t is the number of the learning cycle, i=1, 2, ..., P) arranged in a lattice of D dimensions (D is a positive integer), and the above equation (14) is an equation to update $W^t_i$ to $W^{t+1}_i$ such that each neuron vector has a similar structure to structures of Ni input vectors $x^t_n(S_i)$ classified into the neuron vector].

**21.** The recording medium according to claim 19, wherein the program is a program for performing processing of the following equations (15), (16) and (17):

$$W^0_{ij} = x_{ave} + 5\sigma_I \left\{ b_I \left[ \frac{i - I/2}{I} \right] + b_2 \left[ \frac{j - J/2}{J} \right] \right\} \tag{15}$$

[in which, $W^0_{ij}$ represents P (P=I×J) initial neuron vectors arranged in a two-dimensional (*i,j*) lattice (*i*=1, 2, ..., *I,j* = 1, 2, ..., *J*), $x_{ave}$ is the average value of K (K is a positive integer of 3 or above) input vectors {$x_1$, $x_2$, ..., $x_K$} of M dimensions (M is a positive integer), $b_1$ and $b_2$ are the first principal component vector and the second principal component vector respectively obtained by principal component analysis on the input vectors {$x_1$, $x_2$, ..., $x_K$}, and $\sigma_1$ is the standard deviation of the first principal component of the input vectors]

$$W^{t+1}{}_{ij} = W^t{}_{ij} + \alpha(t)\left[ \frac{\displaystyle\sum_{x_k \in S_{ij}} x_k}{N_{ij}} - W^t{}_{ij} \right] \tag{16}$$

$$\alpha(t) = \max\left\{ 0.01,\, 0.6\left[ 1 - \frac{t}{T} \right] \right\} \tag{17}$$

[in which, $W^t{}_{ij}$ represents P (P=I×J) initial neuron vectors (t is the number of learning cycle, t= 1, 2, ..., T) arranged in a two-dimensional (*i,j*) lattice (*i*=1, 2, ..., *I, j* = 1, 2, ..., *J*), and the above equation (16) is an equation to update $W^t{}_{ij}$ to $W^{t+1}{}_{ij}$ such that each neuron vector has a similar structure to structures of input vectors classified into the neuron vector and $N_{ij}$ input vectors $x^t{}_n(S_{ij})$ classified into the neighborhood of the neuron vector; the term $\alpha(t)$ denotes a learning coefficient ($0<\alpha(t)<1$) for the t-th epoch when the number of learning cycles is set to T epochs, and is expressed using a monotone decreasing function.].

22. The recording medium according to any one of claims 12 to 21, wherein the recording medium is a recording medium selected from floppy disk, hard disk, magnetic tape, CD-ROM, CD-R, CD-RW, DVD-ROM, DVD-RAM and DVD-RW.

23. A computer based system using the computer readable recording medium according to any one of claims 12 to 22.

# FIG. 1

(STEP 1)

INPUT INPUT VECTER DATA TO A COMPUTER

(STEP 2)

SET UP INITIAL NEURON VECTOR $W^0$

(STEP 3)

CLASSIFY INPUT VECTOR $x_k$ INTO $W^t_{p'}$
(PARALLEL PROCESSING POSSIBLE)

(STEP 4)

(STEP 5)

UPDATE $W^t_{p'}$
(PARALLEL PROCESSING POSSIBLE)

NO

LEARNING
CYCLE t EQUALS PRESET
NUMBER T?

YES

(STEP 6)

CLASSIFY $x_k$ INTO $W^T_{p'}$
(PARALLEL PROCESSING POSSIBLE)

EP 1 248 230 A1

# Fig. 2

Fig. 3A

Fig. 3B

# Fig. 4

Fig. 5A

Fig. 5B

EP 1 248 230 A1

Fig. 6

# Fig. 7A

# Fig. 7B

# Fig. 7C

## INTERNATIONAL SEARCH REPORT

| International application No. |
|---|
| PCT/JP00/08666 |

**A. CLASSIFICATION OF SUBJECT MATTER**
Int.Cl$^7$ G06N3/00

According to International Patent Classification (IPC) or to both national classification and IPC

**B. FIELDS SEARCHED**

Minimum documentation searched (classification system followed by classification symbols)
Int.Cl$^7$ G06N3/00

Documentation searched other than minimum documentation to the extent that such documents are included in the fields searched
Jitsuyo Shinan Koho          1926-1996      Jitsuyo Shinan Toroku Koho  1996-2001
Kokai Jitsuyo Shinan Koho  1971-2001      Toroku Jitsuyo Shinan Koho  1994-2001

Electronic data base consulted during the international search (name of data base and, where practicable, search terms used)
INSPEC                                (Kohonen * multivariate)
JICST DATA BASE (JOIS) (KOHONEN*NEURAL NETWORK*MULTIVARIATE ANALYSIS)
(in Japanese)

**C. DOCUMENTS CONSIDERED TO BE RELEVANT**

| Category* | Citation of document, with indication, where appropriate, of the relevant passages | Relevant to claim No. |
|---|---|---|
| X<br>A | IEEE TRANSACTIONS ON NEURAL NETWORKS, Vol.11, No.3,<br>31 May, 2000 (31.05.00), Mu-Chun Su, Hsiao-Te Chang,<br>"Fast Self-Organizing Feature Map Algorithm", p.721-p.733 | 1,2,5,12,16,20<br>,23,24<br>3,4,6-11,<br>13-15,17-19,21<br>,22 |
| A | IEEE TRANSACTIONS ON NEURAL NETWORKS, Vol.11, No.3,<br>31 May, 2000 (31.05.00), Juha Vesanto,<br>Esa Alhoniemi, "Clustering of the Self-Organizing Map",<br>p.586-p.600 | 1-24 |
| A | JP, 4-195247, A (Toshiba Corporation),<br>15 July, 1992 (15.07.92),<br>Claims | 1-24 |
| A | JP, 9-288656, A (NEC Corporation),<br>04 November, 1997 (04.11.97),<br>Full text | 1-24 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>05 March, 2001 (05.03.01) | Date of mailing of the international search report<br>21 March, 2001 (21.03.01) |
|---|---|
| Name and mailing address of the ISA/<br>Japanese Patent Office | Authorized officer |
| Facsimile No. | Telephone No. |

Form PCT/ISA/210 (second sheet) (July 1992)